(19) Europäisches Patentamt
European Patent Office
Office européen des brevets

(11) EP 3 216 489 B1

(12) EUROPEAN PATENT SPECIFICATION

(45) Date of publication and mention of the grant of the patent:
31.10.2018 Bulletin 2018/44

(51) Int Cl.:
A61N 1/36 (2006.01)

(21) Application number: 17159833.7

(22) Date of filing: 08.03.2017

(54) **SYSTEMS AND METHODS FOR AUTOMATED CHARGE BALANCING OF MULTIPLE ELECTRODES FOR UNINTERRUPTED THERAPY AND EVOKED RESPONSE SENSING**

SYSTEME UND VERFAHREN FÜR AUTOMATISIERTEN LADUNGSAUSGLEICH MEHRERER ELEKTRODEN FÜR UNTERBRECHUNGSFREIE THERAPIE UND EVOZIERTE REAKTIONSERFASSUNG

SYSTÈMES ET PROCÉDÉS AUTOMATISÉS D'ÉQUILIBRAGE DE CHARGE DE PLUSIEURS ÉLECTRODES POUR UNE THÉRAPIE ININTERROMPUE ET DÉTECTION DE RÉPONSE ÉVOQUÉE

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 10.03.2016 US 201662306093 P

(43) Date of publication of application:
13.09.2017 Bulletin 2017/37

(73) Proprietor: BIOTRONIK SE & Co. KG
12359 Berlin (DE)

(72) Inventors:
• Baru, Marcelo
Tualatin, OR 97062 (US)
• Vijayagopal, Ramprasad
Sugar Land, TX 77479 (US)

(74) Representative: Keck, Hans-Georg
BIOTRONIK SE & Co. KG
Corporate Intellectual Properties
Woermannkehre 1
12359 Berlin (DE)

(56) References cited:
WO-A1-97/21324          WO-A1-2014/134075
WO-A1-2015/164418     US-A1- 2011 125 217

• ANH TUAN DO ET AL: "A current-mode stimulator circuit with two-step charge balancing background calibration", CIRCUITS AND SYSTEMS (ISCAS), 2013 IEEE INTERNATIONAL SYMPOSIUM ON, IEEE, 19 May 2013 (2013-05-19), pages 409-412, XP032445942, DOI: 10.1109/ISCAS.2013.6571867 ISBN: 978-1-4673-5760-9

## Description

The invention relates to systems and methods for automatic determination of charge balancing during electrical stimulation of a target using a pulse generator having at least one stimulating electrode, at least one return electrode, and at least one forced return electrode when two or more return electrodes are utilized for therapy.

Upcoming neurostimulation applications (e.g. implantable) are demanding pulse generator architectures that can deal with multiple electrodes active at the same time, inject large currents and support high pulsing rates, with reduced electrode areas (to improve selectivity) and without interruption of stimulation.

These constraints require systems and methods that can utilize information about the charge injection process to transparently modify stimulation patterns in order to avoid excessive electrode potential excursions and deal with mismatches between stimulating and return currents (used for active charge balancing) in order to avoid runaway issues in the DC blocking capacitors in series with each electrode. Considering the charge injection process also permit minimizing the stimulus artifact (SA) for robust sensing of evoked responses (caused by electrical stimulation) enabling future closed-loop neurostimulation applications.

Traditionally programmed charge-balanced stimulation, i.e. the charge injected in a stimulation pulse equals that of a balancing pulse, assumes only reversible chemical reactions occur during therapy delivery which may not be the case. For unipolar cathodic stimulation, E. Hudak proposed in "Electrochemical Evaluation of Platinum and Diamond Electrodes for Neural Stimulation, PhD Thesis, Dept. of Chemical Engineering, Case Western University, May 2011" placing a large value resistor across the DC blocking capacitor in series with the stimulating electrode to bleed off a portion of the stimulation charge forcing the balancing anodic phase to have less charge, in an attempt to compensate for irreversible chemical reactions that may occur during therapy.

However, DC blocking capacitors are an important safety feature in the design of (e.g. implantable) pulse generators. They are primarily placed to limit the charge per phase, reduce DC leakage and prevent DC from circulating through tissue under a fault condition, among other functions. Although various approaches have been proposed in prior art for their elimination, given their typical sizes, reduced safety remains a concern and the reason why they continue to be employed in the design of pulse generators for chronic human implants. Thus, placing a resistor across a DC blocking capacitor as proposed by Hudak is in contrast to the main purposes of the latter component and may therefore impact compliance with tight DC-leakage requirements (e.g. 100 nA maximum) of clinical implantable active devices. Moreover, the compensation proposed by Hudak is open loop causing the shift in electrode potential to be either positive or negative of the open circuit potential (OCP) depending on the amount of imbalance generated by the charge bleeding.

Further, US 6,301,505 B1 discloses an electrical tissue stimulating device which includes circuitry for monitoring the build-up of undesirable residual voltages between stimulation electrodes and reducing such voltages in the event the condition occurs.

Further, US 2011/0125217 A1 also discloses measuring any residual charge remaining in an electrode that may result from an imbalance in the applied stimulation. In particular US 2011/0125217 A1 discloses measuring the voltage across a DC blocking capacitor in series with an electrode to provide an accurate representation of the integral of the charge flow to the electrode and accordingly provide a measure of the residual charge on the electrode contact.

Further, also US 2008/0015641 A1 discloses electrical stimulation via electrodes, wherein the presence of a residual charge at the electrodes is measured via a differential voltage measurement and respective actions are taken in case thresholds are crossed.

WO 2014/134075 A1 teaches an electrical charge balancing method for functional stimulation using precision pulse width compensation.

WO 97/21324 A1 describes a feedback system to control electrode voltages in a cochlear stimulator.

WO 2015/164418 A1 describes a system and method for electrical pulse charge compensation for implantable medical device capacitance loading effects.

A. T. Do et al. describe a current-mode stimulator circuit with two-step charge balancing background calibration (A. T. Do, Y. S. Tan, G. M. Xiong, C. Choong, Z. H. Kong and K. S. Yeo, "A current-mode stimulator circuit with two-step charge balancing background calibration," 2013 IEEE International Symposium on Circuits and Systems (ISCAS2013), Beijing, 2013, pp. 409-412)

Based on the above, the problem underlying the present invention is to provide methods and systems of the afore-mentioned kind that allows automated charge balancing in a safe manner and in particular reduces consumption overhead associated with said balancing.

This problem is solved by a method according to claim 1. Preferred embodiments are stated in the sub claims and are described below.

According to claim 1, the method for automatic charge balancing during electrical stimulation of a target uses a pulse generator (which may be an implantable pulse generator in all embodiments of the present invention) having at least one stimulating electrode (W, X), at least one return electrode (Y), and at least one forced return electrode (Z) when two or more return electrodes are used, wherein the at least one stimulation electrode (W, X) and the at least one return electrode (Y) are configured to deliver electrical stimulation therapy, wherein each electrode (W, X, Y, Z) is coupled via a DC-blocking capacitor ($C_i$) to a current source (S), a current sink (S'), or a voltage, and where each electrode forms a capacitance ($C_{dli}$) when forming a double layer with adjacent target material, wherein

- in a determination stage, programmed stimulation current pulses ($I_{Ni}$) and automatically-determined balancing current pulses ($I_{Pi}$) for the respective electrode (i = W, X, Y, Z) are established such that the difference between the circulating balancing current pulse ($I_{Pi}$) minus the respective stimulation current pulse ($I_{Ni}$) through the respective electrode "i" satisfy:

   - for the at least one stimulating electrode (W, X), the difference equals an automatically determined positive value;
   - for the at least one return electrode (Y) and the at least one forced return electrode (Z), the difference is positive and smaller or equal than the minimum among the difference values for the stimulating electrodes (W, X);

      - for each electrode (i = W, X, Y, Z), both its associated DC-blocking capacitor ($C_i$) and the associated capacitance ($C_{dli}$) charge in the same direction and opposite for stimulating and returning electrodes, and wherein

         - in a stimulation stage succeeding said determination stage, programmed stimulation current pulses followed by automatically-determined balancing current pulses are repeatedly applied via the at least one stimulating electrode (W, X) and at least one return electrode (Y), wherein between a balancing current pulse and the next stimulation current pulse an open circuit phase is conducted where no current is imposed via the at least one stimulating electrode (W, X), and wherein

- in said stimulation stage at least one of the electrodes (i = W, X, Y, Z) is monitored, wherein, when an accumulated voltage ($\Delta Vdli$) at the double layer of the at least one monitored electrode crosses pre-defined thresholds ($-\Delta VAddOCP$, $\Delta VSubOCP$), correction currents (ICORRStim, ICORRRet) are generated that reduce or cancel said accumulated voltages ($\Delta V_{dli}$) (including also all others that did not reach a threshold).

The preferred method (and system) of the present invention thus allow an uninterrupted multi-electrode, controlled charge-imbalanced stimulation that maintains safe voltages at each active electrode while avoiding runaway in the DC blocking capacitors caused by mismatches in the current drivers.

In a preferred embodiment of the method according to the present invention, the current sources, sinks, or voltages are coupled to the respective electrode "i" via a DC-blocking capacitor $C_i$. This means in particular that there is no DC path between the current sources, sinks, or voltages and the respective electrode.

According to another preferred embodiment of the method according to the present invention, crossing of the safety threshold ($-\Delta V_{AddOCP}$, $\Delta V_{SubOCP}$) by said accumulated double layer voltage ($\Delta V_{dli}$) in electrode "i" is automatically detected during therapy delivery by comparing (after P stimulating stages) the voltage on the terminal of the DC-blocking capacitor $C_i$ opposite to the electrode under consideration (i.e. either voltages $V^*_{MUXStim}$, $V^*_{MUXRet}$, or $V^*_{MUXFor}$ depending on whether electrode "i" is a stimulating, a return, or a forced electrode respectively), against the difference between an internally-generated voltage reference (either voltage $V_{REFstim}$, $V_{REFRet}$, or $V_{REFFor}$ respectively depending on the electrode role) and the estimated accumulated voltage at the DC blocking capacitor $C_i$, i.e. either voltage $P\Delta V_{CStim}|^{Per\ Pulse}$, $P\Delta V_{CRet}|^{Per\ Pulse}$, or $P\Delta V_{CForl}|^{Per\ Pulse}$ where the $\Delta V$s are determined during the determination stage.

According to a preferred embodiment of the method of the present invention, crossing of the thresholds ($-\Delta V_{AddOCP}$, $\Delta V_{SubOCP}$) by said accumulated double layer voltage ($\Delta V_{dli}$) is automatically detected by comparing the voltage on the terminal of the DC-blocking capacitor ($C_i$) opposite to the electrode against the difference between an internally-generated voltage reference and an estimated accumulated voltage ($P\Delta V_{CStim}|^{Per\ Pulse}$, $P\Delta V_{CRet}|^{Per\ Pulse}$, or $P\Delta V_{CForl}|^{Per\ Pulse}$) at the DC blocking capacitor ($C_i$).

In another preferred embodiment of the method of the present invention the automatically-determining of balancing current pulses is performed for different patient postures and/or depending on the stimulation frequency.

Preferably, said current difference representing said (necessary) charge imbalance is a minimal current difference that ensures that at each electrode both its associated DC blocking capacitor and the associated double layer capacitance charge in the same direction and opposite for stimulating and return electrodes.

Further, preferably, during said determination stage, parameters that measure the final programmed unbalance for each active electrode are stored. Further, preferably, the stimulating and return electrodes with the largest voltage drift, as well as the forced return electrode (if required), are selected for (indirect) monitoring during the stimulation stage. Alternatively, all electrodes may be (indirectly) monitored.

According to a further embodiment of the method according to the present invention, said current difference (i.e. the necessary charge imbalance) is determined for different patient postures and/or depending on the stimulation frequency (i.e. the frequency of the stimulation stages applied via the electrodes).

Further, according to an embodiment of the method according to the present invention, automatically determining said current difference involves letting each stimulating electrode (e.g. the at least one simulating electrode) and each return electrode but the forced one (which is forced to handle the current mismatches) stimulate against a (pseudo) reference electrode forming a return electrode in the determination stage, wherein particularly said reference electrode is formed by a casing 201 of the pulse generator.

Preferably, the automatically-determining of balancing current pulses of the method according to the present invention involves letting each electrode for delivering electrical stimulation therapy stimulate against a reference electrode, wherein particularly said reference electrode is formed by a casing 201 of the pulse generator.

Said automatically comparing during therapy is preferably conducted by means of a respective comparator which triggers when said threshold is crossed such that said correction currents are injected, particularly for moving the accumulated charges in the opposite direction. These correction phases can either be performed by having a separate active phase during part of the open circuit phases or by adjusting successive balance phases (i.e. balancing current pulses).

Electrical stimulation depolarizes fibers generating propagating action potentials. Evoked compound action potentials (ECAPs) are the sum of electroneurographic (ENG) activity recorded from a number of nerve fibers when these are stimulated above threshold. ECAPs have been used for decades to non-invasively or acutely assess nerve conduction, spinal cord integrity, and cochlear implant fitting among others.

ECAPs amplitudes are typically in the tens of microvolt range and their traditional recording plagued with inherent electrical and other interfering signals orders of magnitude larger. The stimulus artifact (SA) for example, i.e. the non-propagating voltage transient produced as a result of electrical stimulation, is coherent with the ECAP signature and thus cannot be reduced by averaging. Its amplitude may not only saturate the ECAP recording front-end but its effect may extend beyond the duration of the stimulus pulse when the ECAP signature is to be recorded. Interference by much larger electromyographic (EMG) activity of nearby muscles and heart activity (ECG) may also affect ECAP recording.

However, the present invention discloses multi-electrode systems and methods that optimize biphasic electrical stimulation to return the electrode potentials post stimulation close to their open circuit potentials (OCPs). This particularly allows a reduction of SA. In another preferred embodiment, systems and methods according to the invention are therefore preferably also used for measuring ECAPs using a tripolar or quasi tripolar arrangement of three recording electrodes which minimize the effect of interfering signals (remnant SA, EMG, and ECG). Therefore, in a preferred embodiment of the method according to the present invention, the automatically-determining of balancing current pulses is performed such that the stimulus artifact (SA) of a biphasic pulse for evoked compound action potential (ECAP) sensing is minimized.

Particularly, in embodiments using a quasi tripolar arrangement, two outer recording electrodes are tied together and connected to a non-inverting input of a single front-end that is configured for recording said ECAPs whereas a center recording electrode being arranged between said two outer recording electrodes is connected to the inverting input of said front-end instead.

Alternatively, in embodiments using a (true) tripolar arrangement, two recording front-ends are used, wherein each front-end comprises an output, which outputs are summed to form a common output, wherein two outer recording electrode are each connected to a non-inverting input of an associated front-end, and wherein the inverting inputs of the two front-ends are tied together and connected to a central recording electrode arranged between said outer recording electrodes. Further, a component may be included being configured to compensate for misbalances in the recording impedances to minimize pickup of potential interfering signals (e.g. EMG, ECG). In a preferred embodiment, this component may adjust the gains of said two front-ends via control signals, respectively, by minimizing the energy of the difference between said two outputs of the two front-ends.

Further, according to an embodiment of the method according to the present invention, the pulse generator comprises at least a lead and preferably a second (e.g. percutaneous) lead each extending along each other from a first end of the respective lead (e.g. adjacent to the casing 201 of the pulse generator) to a second end of the respective lead (e.g. remote from said casing 201), wherein the at least one stimulating electrode is arranged on one of said leads and guarded by at least one return electrode, and wherein said recording electrodes are arranged on one of said leads.

In a preferred embodiment, the ECAP recording electrodes and the at least one stimulating electrode are arranged at a distance to each other, particularly so as to minimize the SA. Here, the recording electrodes may be arranged adjacent said second ends and the at least one stimulating electrode closer to said first ends.

Preferably, said afore-described distance is the maximal possible distance in the given configuration of the pulse generator and its electrodes.

Further, according to an embodiment of the method according to the present invention, following a programmable blanking period post stimulus (i.e. after a stimulation current pulse and a succeeding balancing current pulse), intermediate electrodes (i.e. electrodes arranged between the at least one stimulating electrode and the recording electrodes) are connected to a voltage reference $V_{REF}$ (e.g. internally generated by the pulse generator) via at least one or several switches, which allows referencing the body to a common voltage suitable for the ECAP recording front-end operation, and wherein said blanking may be accomplished via said disconnecting.

Further, according to a preferred embodiment of the method according to the present invention, a change in a relative position of the leads with respect to each other (or of two inter-lead electrodes with respect to each other) is detected using post-stimulus latency value changes of two ECAPs. Since the pulse generator controls both the timing of the stimulation current pulse delivery and the sampling of ECAPs, it can stimulate at the first end of the first lead and record at the second end of a second lead, thus maximizing the distance between stimulating and recording electrodes.

Furthermore, according to an embodiment of the method according to the invention, a suitable threshold is defined in an initial ECAP signature and the latency to such threshold automatically calculated. Such latency value directly corresponds to the physical distance between the stimulating and recording electrode sites and it is preferably stored in the pulse generator for future comparisons.

Further, according to an embodiment of the present invention, for detecting said change in relative position, the pulse generator is configured to initiate a further (new) ECAP. Particularly, to determine the new ECAP latency, the output of the ECAP recording front-end (or front-ends) is compared against the threshold defined using the initial ECAP. Particularly, since no subsequent ECAP measurements are required following the initial one, the power and memory required for the purpose of determining relative leads migration is minimized. Further, according to an embodiment of the present method, if the initial and subsequent latency values are within some acceptable pre-defined deviation, then it is automatically assumed that the leads (or said two electrodes, e.g. stimulating and recording electrode(s)) have not migrated relative to each other or that they have migrated an acceptable small amount. An unacceptable deviation may, for example, be defined as an abrupt or significant short-term change from the initial latency value.

Particularly, upon detection of an unacceptable latency deviation, the pulse generator can dynamically alter the stimulation via the electrodes or current steering settings. In addition, or as an alternative, the pulse generator may generate an output signal for notifying a physician of the migration of leads.

According to yet another aspect of the present invention, a system having the feature of claim 11 is disclosed. Preferred embodiments of this system are stated in the corresponding sub claims and are described below. The system is preferably used in a method according to the invention. All features described in conjunction with the method according to the invention may be used to further characterize the system according to the invention which is particularly configured to conduct the individual features / method steps of the method according to the invention. Vice versa, all features described in context with the system according to the present invention may be used to characterize individual steps of the method according to the invention.

As per claim 11, the system according to the present invention for automatic charge balancing during electrical stimulation of a target comprises a pulse generator having at least one stimulating electrode (W, X), at least one return electrode (Y), and at least one forced return electrode (Z) when two or more return electrodes are used, wherein the at least one stimulation electrode (W, X) and the at least one return electrode (Y) are configured to deliver electrical stimulation therapy, wherein each electrode is coupled via a DC-blocking capacitor ($C_i$) to a current source (S), a current sink (S'), or a voltage, and where each electrode forms a capacitance ($C_{dli}$) when forming a double layer with adjacent target material, wherein

- the system is configured to program in a determination stage stimulation current pulses $I_{Ni}$ and automatically-determined balancing current pulses $I_{Pi}$ for the respective electrode (i = W, X, Y, Z) such that the difference between the circulating balancing current pulse ($I_{Pi}$) minus the respective stimulation current pulse ($I_{Ni}$) through the respective electrode satisfy:

  - for the at least one stimulating electrode (W, X), the difference equals an automatically determined positive value;
  - for the at least one return electrode (Y) and the at least one forced return electrode (Z), the difference is positive and smaller or equal than the minimum among the difference values for the stimulating electrodes (W, X);
  - for each electrode (i = W, X, Y, Z), both its associated DC-blocking capacitor ($C_i$) and the associated capacitance ($C_{dli}$) charge in the same direction and opposite for stimulating and returning electrodes, and wherein

    - the system is further configured to repeatedly inject in a stimulation stage succeeding said determination stage, programmed stimulation current pulses followed by automatically-determined balancing current pulses via the at least one stimulating electrode (W, X) and at least one return electrode (Y), wherein between a balancing current pulse and the next stimulation current pulse an open circuit phase is conducted where no current is imposed via the at least one stimulating electrode (W, X), and wherein

the system is configured to monitor in said stimulation stage at least one of the electrodes (i = W, X, Y, Z), wherein, when an accumulated voltage ($\Delta V_{dli}$) at the double layer of the at least one monitored electrode crosses pre-defined thresholds ($-\Delta V_{AddOCP}$, $\Delta V_{SubOCP}$), correction currents ($I_{CORRStim}$, $I_{CORRRet}$) are generated that reduce or cancel said

accumulated voltages ($\Delta V_{dli}$) (including also all others that did not reach a threshold).

According to a preferred embodiment of the system according to the invention, the system is configured to automatically detect crossing of safety thresholds ($-\Delta V_{AddOCP}$, $\Delta V_{SubOCP}$) by said accumulated double layer voltage $\Delta V_{dli}$ in electrode "i" during therapy delivery by comparing (after P stimulating stages) the voltage on the terminal of the DC-blocking capacitor $C_i$ opposite to the electrode under consideration (i.e. either voltages $V^*_{MUXStim}$, $V^*_{MUXRet}$, or $V^*_{MUXFor}$ depending on whether electrode "i" is a stimulating, a return, or a forced electrode respectively), against the difference between an internally-generated voltage reference (either voltage $V_{REFStim}$, $V_{REFRet}$, or $V_{REFFor}$ respectively depending on the electrode role) and the estimated accumulated voltage at the DC blocking capacitor $C_i$, i.e. either voltage $P\Delta V_{CStim}|^{Per\ Pulse}$, $P\Delta V_{CRet}|^{Per\ Pulse}$, or $P\Delta V_{CFor}|^{Per\ Pulse}$ where the AVs are determined during the determination stage.

As already described above, said current difference representing a charge imbalance is a minimal current difference that ensures that at each electrode both its associated DC blocking capacitor and the associated capacitance charge in the same direction and opposite for stimulating and return electrodes.

Further, preferably, the system is configured to store (e.g. during said determination stage) parameters that measure said unbalance for each electrode. Furthermore, in an embodiment, the system may be configured to select for (e.g. indirect) monitoring the stimulation and return electrodes with the largest voltage drift, as well as the forced return electrode. Alternatively, the system may be configured to (indirectly) monitor all electrodes (i.e. stimulating, return, and forced return electrodes) of the system.

Further, according to an embodiment of the system according to the invention, the system is configured to determine said current difference (i.e. the necessary imbalance) for different patient postures and/or depending on the stimulation frequency.

Further, according to an embodiment of the system according to the invention, for determining said current difference, the system is configured to let each stimulating electrode stimulate against a (pseudo) reference electrode forming a return electrode in the determination stage, wherein particularly said reference electrode is formed by a casing 201 of the pulse generator.

Further, according to an embodiment of the system according to the invention, for automatically-determining of balancing current pulses, the system is configured such that the stimulus artifact (SA) of a biphasic pulse for evoked compound action potential (ECAP) sensing is minimized.

Further, according to an embodiment of the system according to the invention, the system further comprises three recording electrodes that are arranged in a quasi tripolar or a tripolar configuration for measuring ECAPs (see also above).

Particularly, as already described above, in said quasi tripolar configuration the system comprises two outer recording electrodes that are tied together and connected to a non-inverting input of a single front-end comprised by the system that is configured for recording said ECAPs whereas a center recording electrode being arranged between said two outer recording electrodes is connected to the inverting input of said front-end instead.

Alternatively, in a (true) tripolar arrangement, the system comprises two recording front-ends, each comprising an output, which outputs tied together, wherein two outer recording electrodes are each connected to a non-inverting input of an associated front-end (of the two front-ends) and wherein the inverting inputs of the two front-ends are tied together and connected to a central recording electrode of the system that is arranged between said outer recording electrodes. Further, the system may comprise a component being configured to compensate for misbalances in the recording impedances to minimize pickup of potential interfering signals (e.g. EMG, ECG). In a preferred embodiment, this component may be configured to adjust the gains of said two front-ends via control signals, respectively, by minimizing the energy of the difference between said two outputs of the two front-ends.

Further, according to an embodiment of the system according to the invention, the pulse generator comprises at least a first and preferably a second (e.g. percutaneous) lead each extending along each other from a first end of the respective lead (e.g. adjacent the casing 201) to a second end of the respective lead (e.g. remote from the casing 201) wherein the at least one stimulating electrode is arranged on one of said leads and guarded by at least one return electrode, and wherein said recording electrodes are arranged on one of said leads.

Further, according to an embodiment of the system according to the invention, the recording electrodes and the at least one stimulating electrode are arranged at a distance to each other, particularly so as to minimize the SA. Particularly, the recording electrodes may be arranged adjacent said second ends and the at least one stimulating electrode closer to said first ends. Particularly, said distance is the maximal possible distance in the given configuration of the pulse generator and its electrodes.

Further, according to an embodiment of the system according to the invention, the system is particularly configured to conduct a programmable blanking period post stimulus (i.e. after a stimulation current pulse and a succeeding balancing current pulse), wherein the system is configured to connect intermediate electrodes (i.e. electrodes arranged between the at least one stimulating electrode and the recording electrodes) to a reference voltage $V_{REF}$ or to disconnect said electrodes from said reference voltage via at least one or several switches, which allows referencing the body to a common voltage suitable for the ECAP recording front-end operation, and wherein the system may be configured to accomplish said blanking via said disconnecting.

Further, according to an embodiment of the system according to the invention, the system is configured to detect a change in a relative position of the leads (if more than one is present) with respect to each other or of two electrodes with respect to each other using post-stimulus latency value changes of two ECAPs.

Furthermore, according to an embodiment of the system according to the invention, the system is configured to utilize adaptive frequencies and/or equivalent-time sampling techniques for recording ECAPs.

Furthermore, according to an embodiment of the system according to the invention, the current sources, sinks, or voltages, are coupled to the respective (stimulating, return, or forced return) electrode "i" merely via the respective DC-blocking capacitor $C_i$. This means in particular, that there is no DC path between the current sources, sinks, or voltages and the respective electrode connected thereto via the associated DC blocking capacitor $C_i$.

Further features, embodiments and advantages of the present invention shall be described with reference to the Figures, wherein

Fig. 1        shows the potential of a stimulating electrode when an active charge-balanced stimulation protocol is used in a high rate pulsing application;

Fig. 2        shows a schematic representation of a front-end of an implantable pulse generator of the system according to the invention;

Fig. 3        shows an example of a stimulation phase and a balance phase of the pulse generator of Fig. 2;

Fig. 4        shows a circuit of the system according to the invention for determining the open circuit potential (OCP);

Fig. 5        shows the circuit of Fig. 4 to measure accumulated voltage in cycled electrode "i" during the determination stage;

Fig. 6        shows an N to 3 multiplexer (MUX) block for measuring voltages (a) and monitoring electrodes (b);

Fig. 7        shows a stimulation and balance phase following the determination stage in the example described;

Figs. 8 and 9    show comparators for indirectly monitoring the accumulated double layer voltages;

Fig. 10       show a correction phase for cancelling accumulated double layer voltages;

Fig. 11       show a compare phase preceding a correction phase;

Fig. 12       shows a comparator for stopping the injection of a correction phase;

Fig. 13       shows a system according to a preferred embodiment of the invention comprising an (e.g. implantable) pulse generator and dual percutaneous leads forming three recording electrodes in a quasi tripolar configuration;

Fig. 14       shows a first order impedance model for three recording electrodes for recording ECAPs;

Fig. 15       shows a true tripolar configuration of recording electrodes;

Fig. 16       shows a system according to the invention for detecting migration of the leads; and

Fig. 17       shows a further principle for determining lead migration using latency of ECAPs.

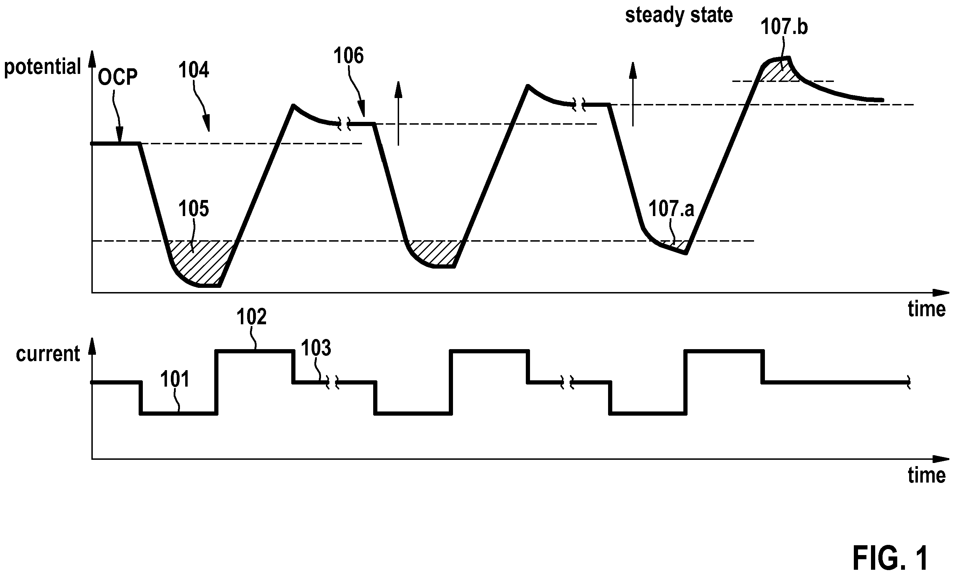

It is a dogma in the field of electrical stimulation that fully-balanced charge-pulses are required for therapy delivery. Figure 1 shows the potential of a stimulating electrode when an active charge-balanced stimulation protocol is used in a high rate pulsing application. In this case, the stimulation pulse consists of a stimulation phase 101 (cathodic pulse) and a balance phase 102 (anodic pulse), followed by an open circuit phase 103 where no current is imposed by the pulse generator. In the following, the pulse generator particularly is an implantable pulse generator (IPG), but the invention also relates to pulse generators in general which may also be non-implantable (e.g. external).

As it can be seen in Figure 1, the electrode potential begins from its open circuit potential (OCP) (measured against a suitable voltage reference electrode). During delivery of the first cathodic pulse 104, the electrode-tissue double layer reversibly charges and the electrode may begin to transfer charge into Faradaic reactions 105 as its potential moves negative. Since it is likely some irreversible charge transfer will occur during the stimulation phase 101, not all of the injected charge may go into charging the double layer under such situation. Hence, only a fraction of the cathodic charge of pulse 104 would be required during the anodic phase 102 to bring the potential back to OCP. If the anodic pulse 102 is balanced with the cathodic one 101 instead, as classically implemented in IPGs, the pre-pulse potential 106 of successive pulses moves positive until the same amount of charge is lost during the cathodic and anodic phases shaded areas 107.a and 107.b. If this occurs, the anodic Faradaic reaction 107.b may cause electrode corrosion. In the case of a platinum (Pt) electrode, for example, Pt oxide (PtO) may be formed and soluble Pt compounds generated when such PtO reacts in the chloride medium. A candidate toxic product is cisplatin $[PtCl_2(NH_3)_2]$.

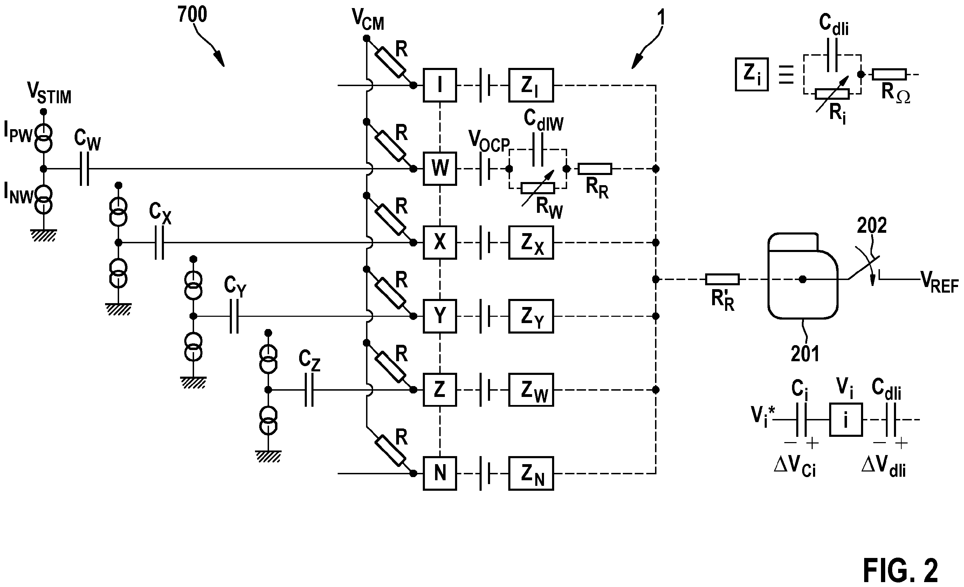

The multi-electrode, multi-current system 1 of Figure 2 according to a preferred embodiment of the present invention is thus particularly configured to deliver stimulation with a scheme that automatically adjusts the charges injected to maintain safe operation, and particularly to prevent voltage runaway in the DC blocking capacitors $C_i$ (where i denotes the electrode, $C_W$, $C_X$, $C_y$, $C_Z$ for electrodes i = W, X, Y, Z are shown without losing generality).

Now, during charge-imbalanced stimulation, the shift in pre-pulse potential may be either positive or negative of the OCP depending on the amount of imbalance. Hence, to be able to monitor electrode voltage drift and compensate for it during therapy (without interruption), the system 1 proposed particularly delivers the minimum charge imbalance necessary that guarantees at each active electrode both its associated DC blocking capacitor $C_i$ and double layer (which are in series) charge in the same direction. Under the proposed system 1 and method, the stimulating electrodes will charge in one direction whereas the return electrodes will charge in the opposite direction to allow compensating when certain voltage limits are reached.

The determination of the necessary imbalance may be performed prior to therapeutical electrical stimulation of the target (e.g. tissue of a patient), for different patient postures, and depending on the stimulation frequency, by first independently cycling through each programmed stimulating electrode (to be used for electrical stimulation) and stimulating (as programmed for electrical stimulation) against a pseudo reference electrode instead. Such pseudo reference may preferably be the IPG case 201. Following, the system 1 cycles through all return electrodes except one which is forced to handle the current mismatches. During this "determination stage", parameters that measure the final programmed "unbalance" for each active electrode are saved, and the stimulation and return electrodes with the largest voltage drift, as well as the forced return electrode, selected for indirect monitoring during the actual electrical stimulation for therapy.

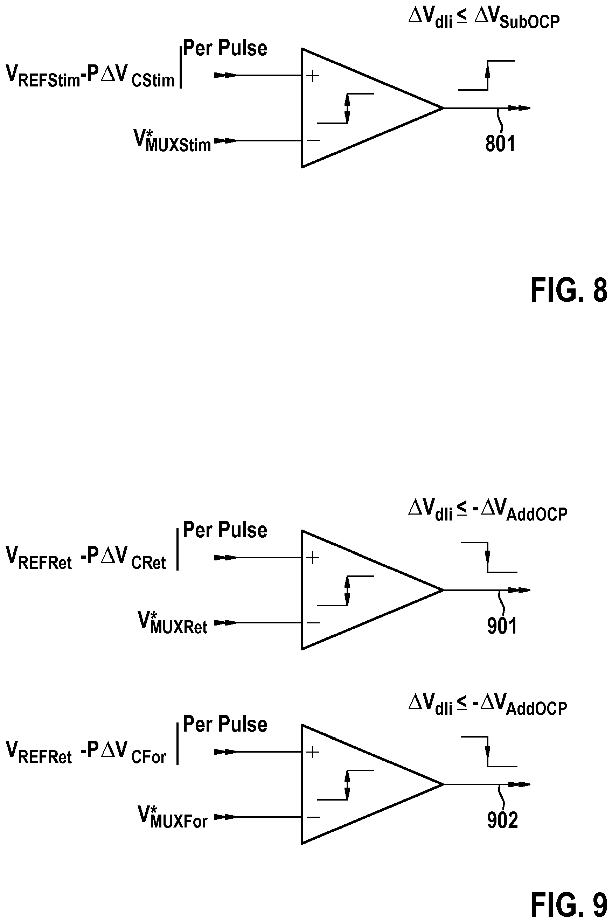

Once the determination stage is completed, electrical stimulation of the target is delivered as programmed and during the open circuit phases the accumulated electrode-tissue double-layer voltages (of the selected electrodes for monitoring) are particularly indirectly compared against variable reference voltages internally generated in the IPG 700. These comparators (cf. e.g. Figs. 8 and 9) preferably allow monitoring the stimulation and return electrode voltages (with the largest excursions), and the forced return electrode, between programmable limits without direct accessing such electrode voltages. Particularly, unlike in prior art, there are no measurements during electrical stimulation of the target (therapy), only comparison of voltages (e.g. a minimum of three voltages on the other side of the DC blocking capacitors for the selected electrodes) that indirectly assess the double-layer voltages accumulated. Advantageously, this approach reduces power consumption (no amplifiers are used during the actual electrical stimulation of the target), minimizes time to decide on the status of the electrodes, and requires no DC path from an electrode.

Particularly, in an embodiment, once a comparator triggers, correction phases take place to start moving the accumulated charges in the opposite direction. Particularly, these correction phases can either be performed by having a separate active phase during part of the open circuit phases or by adjusting successive balance phases.

In the following best modes for carrying out the invention are described in detail. Figure 2 shows a schematic representation of a front-end of a classical multi-electrode (N electrodes) implantable pulse generator (IPG) 700, which may be configured in an embodiment as an IPG of a spinal cord stimulator (SCS) or another application where current steering is desired such as nerve cuff stimulation of peripheral nerves. Particularly, each electrode-tissue interface is modelled by an impedance $Z_i$ (Figure 2 top right) composed of the parallel of the electrode-tissue double-layer capacitance $C_{dli}$ with a variable resistor R; (representative of Faradaic reactions that may occur during stimulation/balancing), in series with $R_\Omega$ which represents the ohmic drop of the tissue electrolyte in the vicinity of an electrode.

Particularly, the IPG case 201 is made of a material that approximates a pseudo reference electrode (e.g. fractal Ir or TiN) and may comprise an effective area that makes its double-layer capacitance $C_{Case}$ (not shown) much larger than $C_{dli}$ (i = 1..N). The electrodes can be made, for example, of Pt, Pt/Ir, or fractal Ir. The open circuit potential (OCP) $V_{OCP}$ shown in Figure 2 is against the IPG case 201 when the latter is connected to an internally-generated voltage reference $V_{REF}$ via switch 202. Since all electrodes are of the same material and have similar areas, it can be considered they all have the same $V_{OCP}$ as reflected in Figure 2.

A similar $R'_\Omega$ represents the ohmic drop in the vicinity of the IPG case 201. The $R_\Omega$ and $R'_\Omega$ actual values are irrelevant as voltage monitoring for safe operation particularly occurs during the open circuit phases 103 when no current is imposed by the IPG 700 (or it can be neglected for the purpose of the analysis). The voltage $V_{STIM}$ in Figure 2 is particularly programmed in an embodiment with the required minimum overhead for steady-state stimulation.

$C_i$ represent the DC blocking capacitor associated with each electrode (i = 1..N, only $C_W$ to $C_Z$ shown in Figure 2) which are nominally all equal. It can also be guaranteed by design that $C_{case}$ results much larger than $C_i$. Typically, the $C_i$ value used in IPGs is in the order of 10 $\mu$F. $C_{dli}$, on the other hand, for an SCS Pt electrode, for example, has a value in the order of 12.5 $\mu$F. Fractal Ir coated electrodes will present a higher $C_{dli}$. In the case of nerve cuff electrodes, $C_{dli}$ may be lower. The present invention, however, assumes no particular relative values between $C_{dli}$ and $C_i$ for the purpose of implementing safe electrical stimulation.

Components R in Figure 2 are bleeding resistors (e.g. hundreds of k$\Omega$), placed in star configuration, typically utilized in IPG's front-ends for passive charge neutrality. In a preferred embodiment, the present invention re-utilizes such network for the purpose of implementing safe stimulation as it will be described below. The IPG case 201 may also have a bleeding resistor connected to it which would slightly alter the calculations shown below.

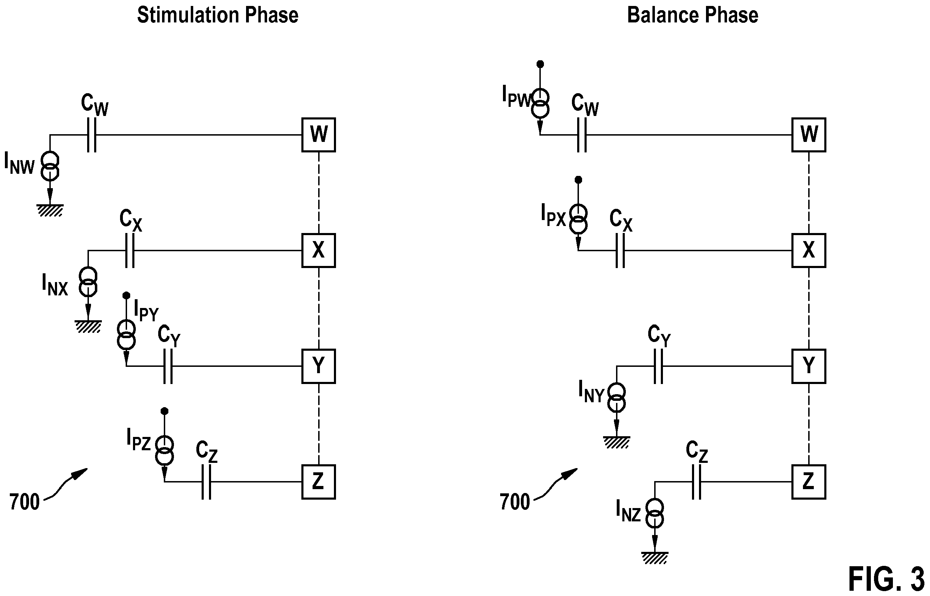

It is now assumed without losing generality that electrodes W, X, Y, Z are active during delivery of electrical stimulation to the target and that, for example, W, X are the stimulating electrodes and Y, Z the return electrodes of the stimulation phases as shown in Figure 3. During the stimulation phase, sinking currents $I_{NW}$ and $I_{NX}$ will flow through electrodes W and X respectively, whereas sourcing currents $I_{PY}$ and $I_{PZ}$ flow through electrodes Y and Z. Currents are particularly programmed so the total cathodic current equals the total anodic current, i.e. in this case

$$I_{NW} + I_{NX} = I_{PY} + I_{PZ} \qquad (1)$$

Assuming the sourcing currents (those from $V_{STIM}$) present larger output impedance than the sinking ones (those to ground or another voltage reference), the latter will accommodate their real values to satisfy eq. (1). In a preferred embodiment, the system of the present invention adjusts the output impedance of the current source associated with at least one of the return electrodes in the stimulation phase (e.g. contact Z) to implement tissue and electrode safe operation. This is further described later on.

An active balance phase is opposite as shown in Figure 3, i.e. currents $I_{PW}$ and $I_{PX}$ flow instead through electrodes W and X respectively, whereas currents $I_{NY}$ and $I_{NZ}$ will flow through electrodes Y and Z.

For the actual electrical stimulation of the target (therapy), currents $I_{NW}$, $I_{NX}$, $I_{PY}$, and $I_{PZ}$, the stimulation phase pulse width ($PW_{Stim}$, common to all), the balance phase pulse width ($PW_{Bal}$, common to all), the inter phases delay (i.e. the time between the end of a stimulation pulse and the start of the associated balancing pulse), and the stimulation frequency are typically selectable and programmable in an IPG 700. For high pulsing rates, and for closed-loop neurostimulation based on neural response, $PW_{Bal}$ is preferably selected equal to $PW_{Stim}$ and programmed as a single parameter pulse width (PW). The balance phase currents $I_{PW}$, $I_{PX}$, $I_{NY}$, and $I_{NZ}$ can be the unknowns the system may adjust to implement safe stimulation without therapy interruption and minimize the stimulation artifact (SA) for evoked response sensing.

Hence, in a preferred embodiment of the present invention, the balance phase currents are automatically determined for safe operation.

For safe tissue and electrode stimulation, the accumulated voltage of the equivalent double-layer capacitances ($\Delta V_{dli}$ where i = W, X, Y, Z in the example) shall remain within a safe window. With the sign shown in Figure 2 (bottom right), this translates into

$$-\Delta V_{AddOCP} \leq \Delta V_{dli} \leq \Delta V_{SubOCP} \qquad (2)$$

where $\Delta V_{SubOCP}$ and $\Delta V_{AddOCP}$ limit the excursion of the electrode voltage in the negative and positive direction with respect to its open circuit potential (OCP) respectively. The limit values may be determined via in-vitro experiments using a suitable electrolyte (and confirmed in-vivo) and programmed in the IPG 700. In a preferred embodiment, the window is symmetrical and a few hundred mV wide (e.g. $\pm$ 100 mV).

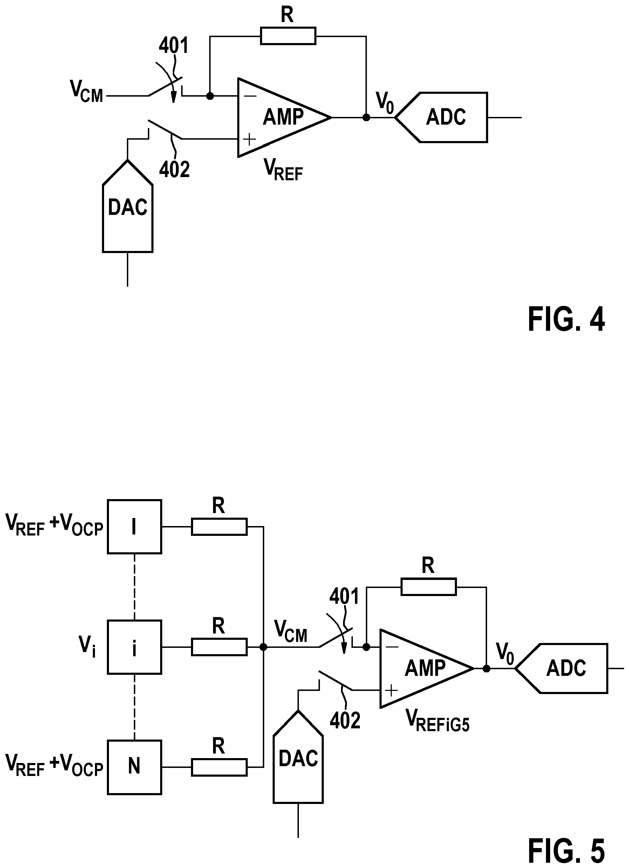

A preferred embodiment for safe stimulation is the following: prior to delivery of the actual electrical stimulation to the target and particularly for different patient postures, the IPG 700 first estimates $V_{OCP}$. To do so, it is configured to measure the common point $V_{CM}$ of the bleeding resistor network R (see Figure 2), preferably via the circuit of Figure 4 (switches 401 and 402 are closed) when the digital-to-analog converter block (DAC) outputs a reference voltage $V_{REF}$ and the IPG case 201 is connected to such voltage. In such case, the output Vo of the amplifier AMP equals

$$Vo = -N\ V_{OCP} + V_{REF} \qquad (3)$$

which is preferably digitized via the analog-to-digital converter block (ADC) and the $V_{OCP}$ calculated and stored in the IPG 700 (N is typically 2, 4, 8, 16, or 32 in a neuro stimulator so digital division is straightforward). Switches 401 and 402 are particularly designed with negligible charge injection and on-resistance compared to R. The amplifier AMP offset is particularly also negligible for the purpose of determining $V_{OCP}$. The resistor R in the feedback of amplifier AMP is preferably matched with the resistors R of Figure 2 to reduce measurement error.

Now, as mentioned before, to be able to monitor voltage drift and compensate for it, the system 1 is preferably configured to deliver the minimum charge imbalance that guarantees (at each electrode) that both $C_i$ and $C_{dli}$ charge in the same direction. The stimulating electrodes (W and X in the example) and the return electrodes (Y and Z in the example) of the stimulation phase will charge in opposite directions to allow compensating once a limit given by conditions (2) is reached.

The determination stage prior to the stimulation stage (where the actual electrical stimulation of the target takes place) may proceed as follows:

The system 1 preferably first cycles through each stimulating electrode independently (W and X in the example) and injects M (M = 2, 4, 8,..) "balanced as-programmed therapy pulses" (i.e. $I_{Pi}$ is automatically programmed by the IPG 700 equal to $I_{Ni}$) against the IPG case 201 (return electrode in the determination stage). The balance will then only be limited by the current matching between the real $I_{Ni}$ and real $I_{Pi}$ (which is typically calibrated for and a few percent apart). M may be selected to improve accuracy of the calculations detailed below. In between the cycling of electrodes W and X (in the example), a complete passive balance phase for electrode W and IPG case 201 (with hardware not shown in Figure 2) is preferably performed to guarantee an electrical-neutral system before cycling the next electrode (X in the example). The same procedure applies when two or more stimulating electrodes are utilized instead for therapy.

For the determination stage, $V_{STIM}$ may be re-programmed with different values to mimic the actual varying voltage that will appear across each current source/sink during therapy.

For electrode W, for example, $V_{STIM}$ may be temporarily re-programmed (during the determination stage) with a value equal to

$$V_{DSn} + R_{W2casemax} * I_{NWmax} + (I_{NWmax} * PW) / C_{WdlWmin}$$

where $V_{DSn}$ is a "safe" compliance voltage required for the current sinks to operate, $R_{W2casemax}$ is the measured impedance between electrode W and the IPG case 201 increased by the measurement error, $I_{NWmax}$ is the stimulation current through electrode W increased by the allowable error, PW is the stimulation pulse width, and $C_{WdlWmin}$ is the measured series capacitor $C_W$, $C_{dlW}$ decreased by the measurement error. It is particularly assumed that $V_{DSn}$ has enough overhead to accommodate the maximum steady-state accumulated voltage on $C_W$ and $C_{dlW}$ for the determination stage to properly operate under such reduced $V_{STIM}$. Given each electrode is much smaller than the IPG case 201, this setup emulates what each electrode will see under a multi-current therapy setup.

After the M determination pulses in the stimulating electrode "i" (i = W or X in the example), connecting again the circuit of Figure 4 and the IPG case 201 results in the circuit of Figure 5. All electrodes, except the cycled "i" (which was active), still present a voltage equal to ($V_{REF} + V_{OCP}$). Programming now the DAC block reference to a voltage $V_{REFFIG5}$ equal to such ($V_{REF} + V_{OCP}$) results in

$$Vo = \Delta V_{dli} + V_{REFFIG5} \qquad (4)$$

as

$$V_i = - \Delta V_{dli} + V_{REF} + V_{OCP} = - \Delta V_{dli} + V_{REFFIG5} \qquad (5)$$

(see Figure 2 for the defined sign of $\Delta V_{dli}$).

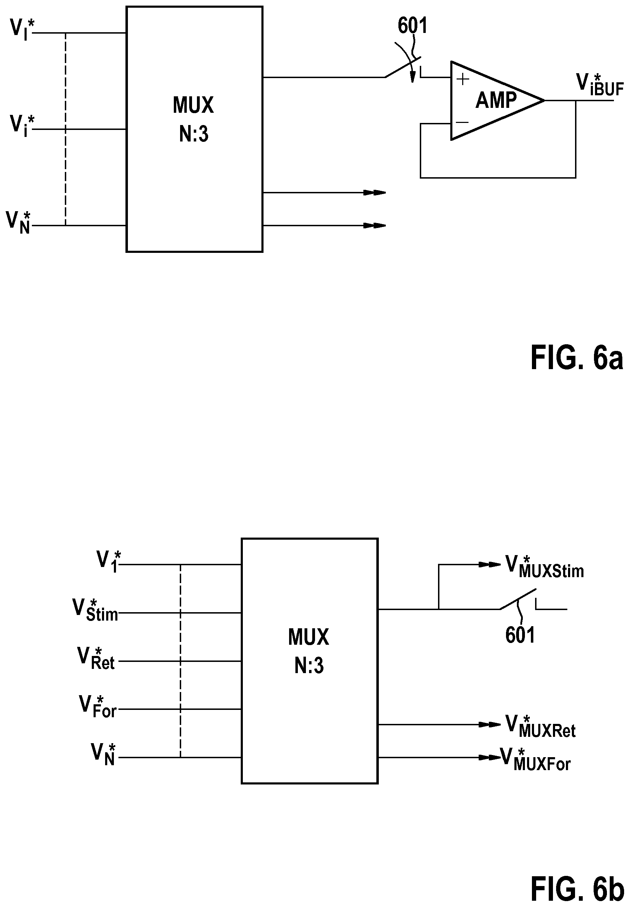

At the same time, the system 1 particularly also measures $V^*_i$ which is the voltage at the other terminal of the DC blocking capacitor $C_i$ of active electrode "i" cycled (see Figure 2 bottom right). This is measured via the N to 3 multiplexer (MUX) block, switch 601 and buffer (AMP) shown in Figure 6.a ($V^*_{iBUF}$ is the output signal).

From $V_i$ determined above (see eq. (5)) and $V^*_{iBUF}$, the accumulated voltage $\Delta V_{Ci}$ (from current mismatches) on the blocking capacitor $C_i$ can be calculated as ($V_i - V^*_{iBUF}$) (see Figure 2 for the sign of $\Delta V_{Ci}$ as measured).

If both $\Delta V_{dli}$ and $\Delta V_{Ci}$ resulted positive, the balance phase for the cycled electrode "i" can be left as programmed for the determination stage. No adjustments are necessary as the positive voltages indicate the mismatch in the real $I_{Ni}$ and real $I_{Pi}$ is causing the balancing charge to be less than the stimulation charge. The misbalance current $I_{Diffi}$, i.e. real

$I_{Ni}$-real $I_{Pi}$, can be estimated to be at least

$$I_{Diffi} = C_{imin} (\Delta V_{Ci}) / (M\ PW) \qquad (i = W\ or\ X\ in\ the\ example) \qquad (6)$$

where $C_{imin}$ is the minimum value of the DC blocking capacitor $C_i$, $\Delta V_{Ci}$ is the above measured accumulated voltage, and PW is the programmed pulse width as defined before.

If $\Delta V_{dli}$ is negative, on the other hand, this implies the electrode "i" potential would be moving positively pulse after pulse so less balancing charge is required to avoid this situation. In a preferred embodiment, the required reduction is determined as follows:

A prior impedance measurement allows estimating $C_{dli}$ for the electrode "i" under consideration (either W or X in the example), with a certain error, so the current $I_{Lessi}$ required to be subtracted from the automatically selected $I_{Pi}$ in this case can be calculated as follows

$$I_{Lessi} = C_{dlimax} (-\Delta V_{dli}) / (M\ PW) \qquad (i = W\ or\ X\ or\ none\ in\ the\ example) \qquad (7)$$

where $C_{dlimax}$ is the measured $C_{dli}$ with the maximum added error, $\Delta V_{dli}$ is the above measured accumulated double-layer voltage (see Figure 2), and PW is the programmed pulse width as defined before.

A lookup table can be implemented in the IPG 700 to determine each $I_{Diffi}$, $I_{Lessi}$ based on the corresponding C, $\Delta V$, and (M PW).

For those electrodes with $\Delta V_{dli}$ negative, $I_{Pi}$ will then be automatically re-programmed equal to

$$new\ I_{Pi} = old\ I_{Pi} - I_{Lessi} \qquad (i = W\ or\ X\ or\ none\ in\ the\ example) \qquad (8)$$

where $I_{Lessi}$ is the current estimated above.

Having a positive $\Delta V_{dli}$ and a negative $\Delta V_{Ci}$ is not possible as the latter implies the automatically programmed $I_{Pi}$ was larger than the selected $I_{Ni}$ (by mismatch) which will always result in a negative $\Delta V_{dli}$ regardless of whether Faradaic reactions were present or not during the stimulation phase.

After initially cycling through all stimulating electrodes, a new set of M pulses, with the modified balance phase, is preferably injected for the stimulating electrodes that required $I_{Pi}$ adjustment, their new $I_{Diffi}$ estimated and stored, and confirming both $C_i$ and $C_{dli}$ accumulated charge in the same direction.

At the end of this process, all stimulating electrodes "i" (W and X in the example) will in theory satisfy

$$real\ I_{Pi} = real\ I_{Ni} - I_{Diffi} \qquad (9)$$

The lowest value among the estimated $I_{Diffi}$ from all stimulating electrodes (W and X in the example) is stored in the IPG 700 as $I_{MinDiff}$. Alternatively, the $\Sigma\ I_{Diffi}$ divided by the number of return electrodes in the stimulation phase is stored instead as $I_{MinDiff}$.

Hence, in this way, $\Delta V_{Ci}$ for the stimulating electrodes (W and X in the example) will have the same positive sign as $\Delta V_{dli}$ as the real $I_{Pi}$ for therapy is guaranteed to be less than $I_{Ni}$.

However, $I_{Pi}$ was determined with only one electrode active. For the same $I_{Pi}$ to flow during therapy where all programmed electrodes are active simultaneously, at least a return electrode in the stimulation phase (e.g. Z considering without losing generality that $I_{PZ}$ is the smallest return current amplitude of the stimulation phase) needs to be forced to present lower impedance than the sinking currents so the $I_{Ni}$ currents get properly established.

On the other hand, in the case of the return electrodes of the stimulation phase, except for the one forced to have lower impedance (i.e. Z in the example), the balance phase currents are preferably automatically programmed equal to

$$I_{Ni} = I_{Pi} - I_{MinDiff} \qquad (i = Y\ in\ the\ example) \qquad (10)$$

where $I_{MinDiff}$ was stored in the IPG 700 as described before.

The system 1 will then preferably cycle independently through each return electrode of the stimulation phase except the forced one (only Y in the example), injecting again M (M = 2, 4, 8,..) with the selected $I_{Pi}$ and automatically-programmed $I_{Ni}$ (see eq. (10)) against the IPG case 201 (return electrode in this stage).

After the M pulses, the difference between the real $I_{Pi}$ and real $I_{Ni}$ can be estimated as follows

$$(\text{real } I_{Pi} - \text{real } I_{Ni}) = C_{imax} (-\Delta V_{Ci}) / (M\ PW) \quad (11)$$

The system 1 then verifies

$$0 < (\text{real } I_{Pi} - \text{real } I_{Ni}) \leq I_{MinDiff} \qquad (12)$$

and (real $I_{Pi}$ - real $I_{Ni}$) is defined as $\Delta I_i$.

If condition (12) is not satisfied, the system 1 can automatically adjust $I_{Ni}$ until condition (12) is satisfied as $I_{Pi}$ is the programmable parameter of the stimulation phase.

The remaining of the sourcing/sinking currents of the stimulation/balance phase will circulate through the forced electrode (Z in the example).

In this way, the stimulating and return electrodes are charging in opposite directions allowing for compensation when one of the conditions (2) is reached.

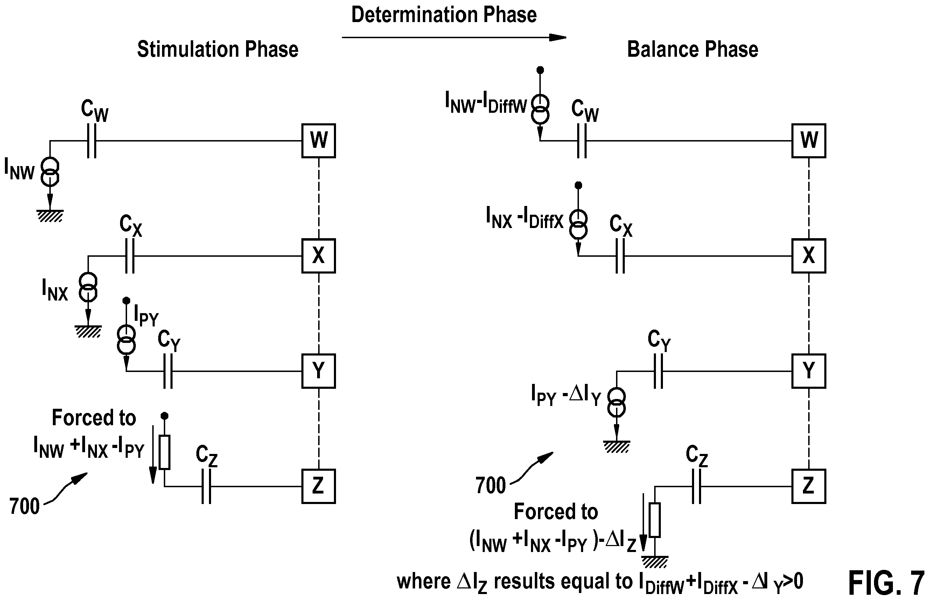

Summarizing, the stimulation and balance phases (post determination stage) in the example being described are shown in Figure 7. Programmable resistors, instead of a current source or sink, can be used to force electrode Z (forming the forced return electrode in the example) to present lower impedance in both the stimulation and balance phases. Considering the stimulation phase, for example, such resistor can be programmed equal to

$$\{V_{SDp} / I_{pZmin} + [(I_{PYmax} / I_{Pzmin}) / C_{YdlYmin} - 1 / C_{ZdlZmax}] * PW + (I_{PYmax} / I_{Pzmin} * R_{Y2allEmax} - R_{Z2allEmin})\}$$

where $V_{SDp}$ is a "safe" compliance voltage required for the current sources to operate, min and max subscripts represent the respective parameters with added or subtracted errors, and $R_{i2allE}$ (i = Y, Z in the example) is the impedance of electrode "i" against all other electrodes tied together. Compliance voltage monitoring across active sink and sourcing currents during the actual electrical stimulation of the target permit confirming the selected resistor is appropriate. If two or more return electrodes are programmed, electrode Z represents the electrode with the smallest programmed current.

As a final step of the determination stage, a new set of M pulses, with the determined balance phase, is preferably injected next for all active electrodes (i.e. both the stimulating and returns) this time, except for the forced one (Z in the example). The parameters $\Delta V_{dli}$ and $\Delta V_{Ci}|^{Per\ Pulse}$ for each electrode are now determined, the latter particularly as the measured $\Delta V_{Ci}/$ M for the selected stimulating and return electrodes, and particularly as

$$[(\Sigma\ I_{Diffi} - \Sigma\ \Delta I_i) * PW] / C_{imin}$$

for the forced electrode (Z in the example), and the former values digitized and stored in the IPG 700. For the forced electrode (Z in the example), a new lookup table can be implemented to determine $\Delta V_{CFor}|^{Per\ Pulse}$ (accumulated per-stimulation pulse voltage in the DC blocking capacitor associated with the forced electrode, Z in the example).

The system 1 will preferably select and monitor (during delivery of the electrical stimulation to the target) the stimulating and return electrodes that presented the largest $|\Delta V_{dli}|$. It will also monitor the forced electrode (Z in the example). The voltages $V^*_{Stim}$, $V^*_{Ret}$, and $V^*_{For}$ (see Figure 6.b) of the selected stimulating, return, and forced electrodes will particularly be connected to $V^*_{MUXStim}$, $V^*_{MUXRet}$, and $V^*_{MUXFor}$ respectively via the MUX. The circuitry of Figure 4 (Figure 5), except for the DAC, and the AMP of Figure 6.a are not needed for the actual electrical stimulation of the target so they may be turned off/disconnected to reduce power consumption.

In an alternative embodiment, all voltages of the participating active electrodes may be monitored instead.

As mentioned before, during electrical stimulation of the target, the system shall guarantee

$$-\Delta V_{AddOCP} \leq \Delta V_{dli} \leq \Delta V_{SubOCP} \quad (i = 1..N)\ (13)\ (\text{same as eq. (2)})$$

Now, during an open circuit phase (no current is imposed by the IPG 700), if the IPG case 201 is connected to $V_{REF}$, in particular one has for the monitored voltages that

$$V_{REF} + V_{OCP} - \Delta V_{dlOutput} - \Delta V_{COutput} - V^*_{MUXOutput} = 0 \qquad (14)$$

(with the sign shown in Figure 2) where Output is either Stim, Ret or For. Eq. (13) can be re-written as

$$\Delta V_{dlOutput} = V_{REF} + V_{OCP} - \Delta V_{COutput} - V^*_{MUXOutput} \qquad (15)$$

At the same time, after P stimulation pulses,

$$\Delta V_{COutput} = \Sigma_{1\ to\ P}\ \Delta V_{COutput}|^{Per\ Pulse} = P\ \Delta V_{COutput}|^{Per\ Pulse} \qquad (16)$$

where the parameter $\Delta V_{COutput}|^{Per\ Pulse}$ was previously digitized and internally stored in the IPG 700 in the final step of the determination stage.
Hence from (13), (15) and (16), for the monitored voltages we have

$$-\Delta V_{AddOCP} \leq V_{REF} + V_{OCP} - P\ \Delta V_{COutput}|^{Per\ Pulse} - V^*_{MUXOutput} \leq \Delta V_{SubOCP} \qquad (17)$$

Conditions (17) can be individually re-written as

$$V^*_{MUXStim} \geq V_{REF} + V_{OCP} - \Delta V_{SubOCP} - P\ \Delta V_{CStim}|^{Per\ Pulse} \qquad (18.a)$$

$$V^*_{MUXRet} \leq V_{REF} + V_{OCP} + \Delta V_{AddOCP} - P\ \Delta V_{CRet}|^{Per\ Pulse} \qquad (18.b)$$

$$V^*_{MUXFor} \leq V_{REF} + V_{OCP} + \Delta V_{AddOCP} - P\ \Delta V_{CFor}|^{Per\ Pulse} \qquad (18.c)$$

It is worth pointing out that $\Delta V_{CRet}|^{Per\ Pulse}$ and $\Delta V_{CFor}|^{Per\ Pulse}$ in conditions 18.b and 18.c are negative so they add to the value on the right of such inequalities.
Conditions 18 can re-written as

$$V^*_{MUXStim} \geq V_{REFStim} - P\ \Delta V_{CStim}|^{Per\ Pulse} \qquad (19.a)$$

$$V^*_{MUXRet} \leq V_{REFRet} - P\ \Delta V_{CRet}|^{Per\ Pulse} \qquad (19.b)$$

$$V^*_{MUXFor} \leq V_{REFRet} - P\ \Delta V_{CFor}|^{Per\ Pulse} \qquad (19.c)$$

where $V_{REFStim}$ and $V_{REFRet}$ are fixed voltages equal to ($V_{REF} + V_{OCP} - \Delta V_{SubOCP}$) and ($V_{REF} + V_{OCP} + \Delta V_{AddOCP}$) respectively.
In a preferred embodiment, condition 19.a is implemented by the comparator of Figure 8 where an extra DAC block generates a variable reference that subtracts the stored $\Delta V_{CStim}|^{Per\ Pulse}$ after each stimulation phase from the internally calculated fixed voltage $V_{REFStim}$, for comparison following the end of the balance phase of pulse P and before the beginning of the next stimulation phase.
Similarly, conditions (19.b) and (19.c) are implemented by the comparators of Figure 9, where a third and fourth internal DAC generate the variable comparison voltages.
If a comparator of Figure 8 or Figure 9 triggers (i.e. outputs 801, 901, or 902 change logic value), after P pulses (a counter is kept in the IPG 700), the corresponding double-layer capacitance and blocking capacitor of the monitored electrode will be discharged.

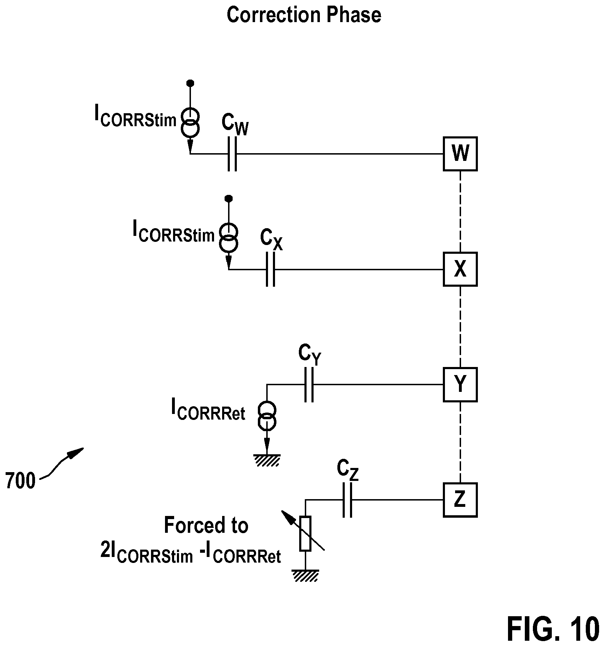

To do so, in a preferred embodiment, a correction phase is implemented as shown in Figure 10 for example. Particularly, a single correction current $I_{CORR}$ will be forced to circulate which will result in real currents $I_{CORRStim}$ for the stimulating electrodes (W and X in the example) and $I_{CORRRet}$ for the return electrodes (only Y in the example). The forced electrode (Z in the example) will handle the difference between the correcting currents.

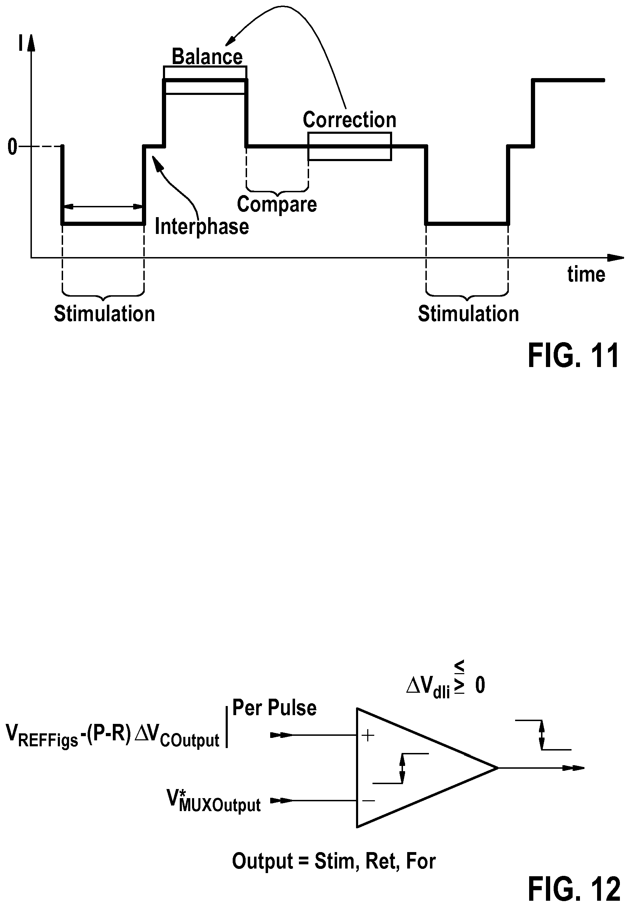

Such correction phases particularly take place following the compare phases (where conditions 18 are evaluated) as shown in Figure 11. It is also possible to stagger the compare and correction phases so they occur in subsequent pulses. In an alternative embodiment, the correction phase is part of the balance phase (e.g. as sketched) where the currents of the latter phase are adjusted accordingly, so as to reduce or cancel the respective accumulated double layer voltage.

In a preferred embodiment, current $I_{CORR}$ is programmed equal to two times $I_{MinDiff}$.

Since it is unknown which capacitor has accumulated more charge, $C_{Output}$ or $C_{dlOutput}$ for the active electrode whose $V^*_{MUXOutput}$ triggered a comparator, the system 1 needs to deliver up to P pulses and stop if $\Delta V_{dlOutput}$ reaches zero voltage ($\Delta V_{COutput}$ will still be positive or negative depending on the electrode). This avoids inverting the charging conditions of the stimulating and return electrodes. Hence, during the injection of the correction phases, the system will make sure the following conditions are satisfied

$$\Delta V_{dlStim} = V_{REF} + V_{OCP} - \Delta V_{CStim} - V^*_{MUXStim} \geq 0 \qquad (20.a)$$

$$\Delta V_{dlRet} = V_{REF} + V_{OCP} - \Delta V_{CRet} - V^*_{MUXRet} \leq 0 \qquad (20.b)$$

$$\Delta V_{dlFor} = V_{REF} + V_{OCP} - \Delta V_{CFor} - V^*_{MUXFor} \leq 0 \qquad (20.c)$$

or re-written as

$$V^*_{MUXStim} \leq V_{REF} + V_{OCP} - \Delta V_{CStim} \qquad (21.a)$$

$$V^*_{MUXRet} \geq V_{REF} + V_{OCP} - \Delta V_{CRet} \qquad (21.b)$$

$$V^*_{MUXFor} \geq V_{REF} + V_{OCP} - \Delta V_{CFor} \qquad (21.c)$$

or re-written as

$$V^*_{MUXStim} \leq V_{REFFIG5} - \Delta V_{CStim} \qquad (22.a)$$

$$V^*_{MUXRet} \geq V_{REFFIG5} - \Delta V_{CRet} \qquad (22.b)$$

$$V^*_{MUXFor} \geq V_{REFFIG5} - \Delta V_{CFor} \qquad (22.c)$$

or re-written as

$$V^*_{MUXStim} \leq V_{REFFIG5} - (P\text{-}R)\, \Delta V_{Cstim}\big|^{Per\ Pulse} \qquad (23.a)$$

$$V^*_{MUXStim} \geq V_{REFFIG5} - (P\text{-}R)\, \Delta V_{CRet}\big|^{Per\ Pulse} \qquad (23.b)$$

$$V^*_{MUXStim} \geq V_{REFFIG5} - (P-R) \left. \Delta V_{CFor} \right|^{Per\ Pulse} \tag{23.c}$$

After R correction phase pulses (R ≤ P), R $\left. \Delta V_{COutput} \right|^{Per\ Pulse}$ has been subtracted from the accumulated $\Delta V_{COutput}$ (given $I_{CORR}$ equals $2I_{MinDiff}$) so $V^*_{MUXOutPut}$ (of the triggered comparator) needs to be compared against a variable reference equal to

$V_{REFFIG5} - (P-R)\Delta V_{COutput}|^{Per\ Pulse}$ as shown in Figure 12.

If the comparator in Figure 12 is triggered, or R equals P, the correction phase is stopped and actual electrical stimulation of the target as per Figure 7 resumed.

Summarizing, the preferred system and method embodiment of the present invention automatically maintains safe electrode and tissue operation particularly without altering the classical IPG 700 front-end thus complying with required safety standards. The use of comparators and not measurements during electrical stimulation of the target (therapy) advantageously minimize overhead consumption for safe operation.

Particularly, when the system 1 is a spinal cord stimulator (SCS), the system and method according to the invention will allow clinically testing a certain multiple-electrode pattern at a tonic frequency (e.g. 40 Hz) that calms pains with associated paresthesia, running a determination stage, and increasing the stimulation frequency to the kHz range to avoid paresthesia. This is not possible with today's SCS products which are limited to two electrodes at the kHz range.

In an alternative preferred embodiment of the present invention, a finer automatic determination of the balance phase permits evoked response sensing, for closed-loop neurostimulation, without the need for a compensatory phase post balance phase or extra circuitry post biphasic stimulation to minimize the stimulus artifact (SA). In this alternative preferred embodiment, if during the stimulation phase $\Delta V_{dli}$ is positive and within $\Delta V_{biph}$, e.g. 5 mV, the balance phase for the cycled electrode "i" can be left as programmed for the determination stage. No adjustments are necessary as the positive voltage indicates the mismatch in the real $I_{Ni}$ and real $I_{Pi}$ is causing the balancing charge to be less than the stimulation charge and the remnant voltage can be handled by the evoked compound action potential (ECAP) recording front-end. In a preferred embodiment, the parameter $\Delta V_{biph}$ is programmable.

If $\Delta V_{dli}$ is positive but larger than $\Delta V_{biph}$, the balancing phase current $I_{Pi}$ needs to be increased for better charge compensation. In a preferred embodiment, the required increase is estimated as follows:
A prior impedance measurement allows determining $C_{dli}$ (with a certain error) for the electrode "i" under consideration (either W or X in the example) so the current $I_{Morei}$ required to be added to the automatically selected $I_{Pi}$ in this case is estimated as

$$I_{Morei} = C_{dli} (\Delta V_{dli} - \Delta V_{biph1/2})/(M\ PW) \quad (i = W\ or\ X) \tag{24}$$

where $\Delta V_{biph1/2}$ is the mid-range point of $\Delta V_{biph}$ and PW is the programmed pulse width as defined before.

A lookup table can be implemented in the IPG 700 to determine each $I_{Morei}$ based on the corresponding $C_{dli}$, ($\Delta V_{dli}$ - $\Delta V_{biph1/2}$) and (M PW).

If $\Delta V_{dli}$ is negative, on the other hand, this implies the electrode potential moved positively pulse after pulse during the M pulses so less balancing charge is required per pulse to avoid this situation. In a preferred embodiment, the required reduction is estimated as follows:

$$I_{Lessi} = C_{dli} (-\Delta V_{dli} + \Delta V_{biph1/2})/(M\ PW)(i = W\ or\ X\ in\ the\ example) \tag{25}$$

The same lookup table for $I_{Morei}$ can be used for determining $I_{Lessi}$.

$I_{Pi}$ will then be re-programmed equal to

$$new\ I_{Pi} = old\ I_{Pi} \pm (I_{Morei}\ or\ I_{Lessi}) \qquad (i = W\ or\ X\ in\ the\ example) \tag{26}$$

After initially cycling through all stimulating electrodes, a new set of M pulses, with the modified balance phase, is injected for the stimulating electrodes that required $I_{Pi}$ adjustment, a new $I_{Diffi}$ then estimated and stored, confirming $\Delta V_{dli}$ is positive and within $\Delta V_{biph}$.
A similar procedure, as detailed before for the determination stage, is followed for the return electrodes and a forced electrode.

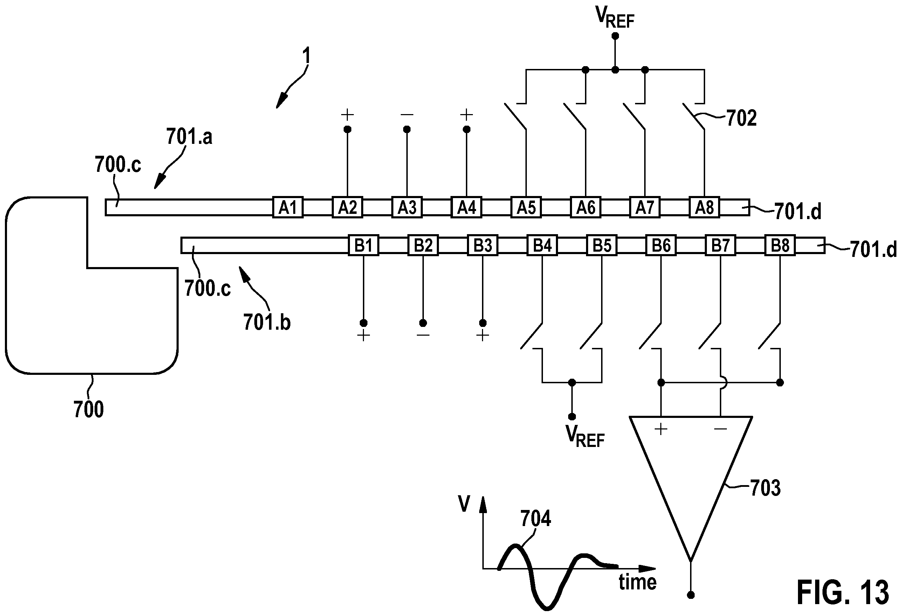

Once the balance phase is automatically determined for the desired stimulation, in a preferred embodiment for evoked response sensing, a quasi tripolar arrangement of electrodes with a body drive is utilized for evoked compound action potential (ECAP) recording. Without losing generality, Figure 13 shows the example of system 1 according to a preferred embodiment of the invention in the form of an implantable SCS system comprising of an IPG 700 and dual percutaneous leads 701.a and 701.b extending each from a first end 701.c to a second (e.g. remote) end 701.d. A typical guarded cathode configuration for stimulation is shown, i.e. A3 and B2 are stimulating electrodes which are surrounded by return electrodes A2, B1, A4 and B3. Sourcing and sinking currents through these electrodes may have different values to steer the electrical field as desired.

Following a programmable blanking period post stimulus, intermediate unused electrodes A5..A8 and B4, B5 (in the example) are connected to a voltage reference $V_{REF}$ (internally generated by the IPG 700) via switches 702 which "drive" the body common mode for recording. Blanking may be accomplished via disconnection of switches 702 and/or other method of placing the ECAP recording front-end 703 in a state so as to minimize the artifactual effect of the blanking termination.

The ends electrodes B6 and B8 (in the example) are tied together and connected to the non-inverting input of the ECAP recording front-end 703 whereas the center electrode B7 (in the example) is connected to the inverting input instead. In a preferred embodiment, the ECAP recording electrodes are selected as far away as possible from the stimulating electrodes to minimize the stimulus artifact (SA). Alternatively, recording can occur using A6 tied to A8 as an electrode and A7 as the other electrode, and A5 and B4..B8 connected to $V_{REF}$.

The recording front-end 703 preferably presents programmable input range and band-pass characteristic, adjustable gain, high input impedance, low equivalent input noise level and power consumption, adequate settling time, high power supply rejection ratio (PSRR), and high common mode rejection ratio (CMRR) among other features.

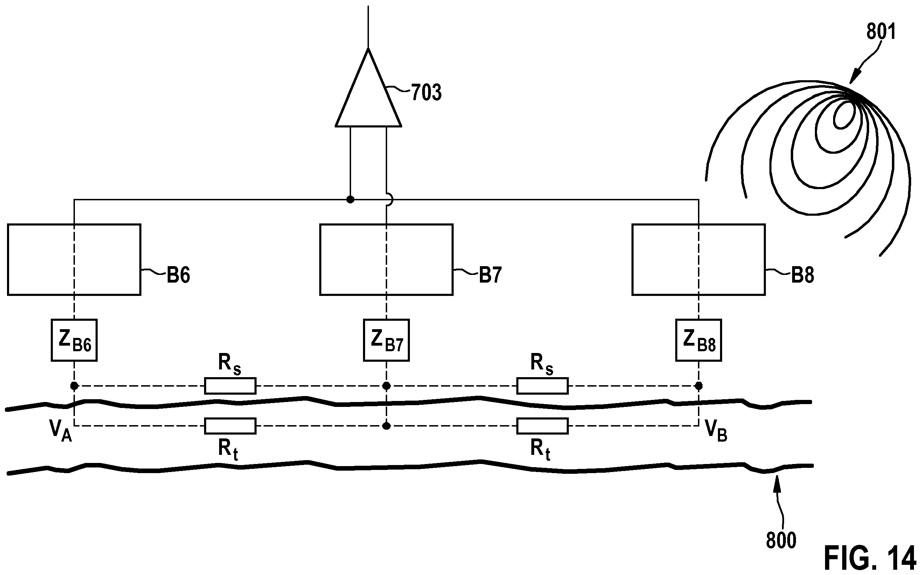

High CMRR allows rejecting electromyographic (EMG) signals of nearby muscles as explained next. Figure 14 shows a first-order impedance model considering the recording electrodes B6..B8 in the example.

Elements $Z_{B6}..Z_{B8}$ model the impedance that exists between each electrode and the fiber bundle 800. Resistive element Rt model the resistance of such fiber bundle 800 where the ECAP 704 (see Figure 13) is travelling and $R_S$ model the shunting presented by the cerebrospinal fluid in the vicinity of the electrodes B6..B8. Let's say an interfering voltage 801 (e.g. EMG and ECG) generates different voltages $V_A$ and $V_B$ in the vicinity of recording electrodes B6 and B8. This would create a voltage $(V_A + V_B)/2$ at electrode B7 given the voltage divider created by Rt//Rs and assuming the recording front-end 703 presents high impedance (i.e. no current circulates through $Z_{B7}$). Given the small distance between B6 and B8, and negligible differences between them (same area and metal), it can be expected $Z_{B6}$ will be similar to $Z_{B8}$. Hence, given they are shunt together, the non-inverting terminal of the recording front-end 703 will also see voltage $(V_A + V_B)/2$. In this way, interfering signal 801 appears as a common mode voltage variation for the ECAP recording front-end 703 which can be suppressed with the appropriate high CMRR.

The recorded ECAP 704 has a triphasic shape as shown in Figure 13 since the quasi tripolar configuration resolves the second derivative of the ECAP 704 with respect to time.

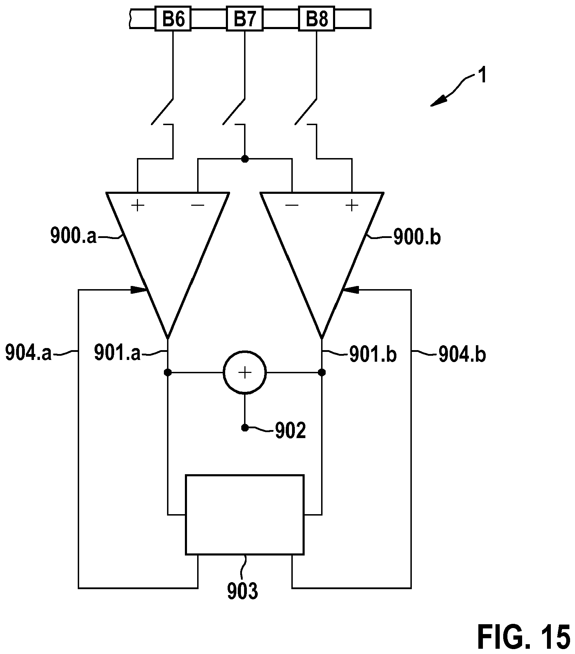

In an alternative embodiment, the recording configuration is a true tripolar one as shown in Figure 15. Two recording front-ends 900.a and 900.b are employed in this case as shown for the example being considered (i.e. B6..B8 as recording electrodes) and their outputs 901.a and 901.b summed to create output 902. Block 903 may be included to compensate for misbalances in the recording impedances (see Figure 14) to minimize pickup of potential interfering signals (e.g. EMG, ECG). In a preferred embodiment, block 903 adjusts the gains of 900.a and 900.b, via control signals 904.a and 904.b respectively, by minimizing the energy of the difference between outputs 901.a and 901.b.

In yet another alternative embodiment, the ECAP recording front-end 703 is switched to a bipolar recording configuration with body drive after several ms post stimulation to observe non-propagating late responses. This late response may allow identifying whether unwanted activation of the nociceptive reflex arc, or muscle afferents in the dorsal roots, is caused by the programmed therapy.

In yet another preferred embodiment, adaptive sampling frequencies may be utilized to record ECAPs and late responses.

In yet another preferred embodiment, ECAPs' signal processing involves a morphological filter algorithm given the reduced signal-to-noise ratio. US 8,419,645 B2 describes how morphological operators can be utilized to determine respiration parameters from a transthoracic impedance signal. These operators can be applied in an analogous manner to process ECAPs 704. In an alternative embodiment, ECAPs signal processing is based on discrete wavelet transforms.

In a further preferred embodiment, the IPG 700 of the present invention senses -ECG via electrodes on leads 701.a, 701.b or electrode(s) and the IPG case 201. Upon detection of an R-wave from such signal, and following a programmable delay (e.g. 300-500 ms), ECAP recording may take place in the refractory period of the cardiac cycle to further reduce pickup of heart activity.

In a further preferred embodiment, ECAP recording may be initiated in combination with a patient posture change automatically detected by circuitry in the IPG 700, a detected electrode-tissue impedance change, manually initiated, or initiated in response to patient triggered adjustments via a remote control.

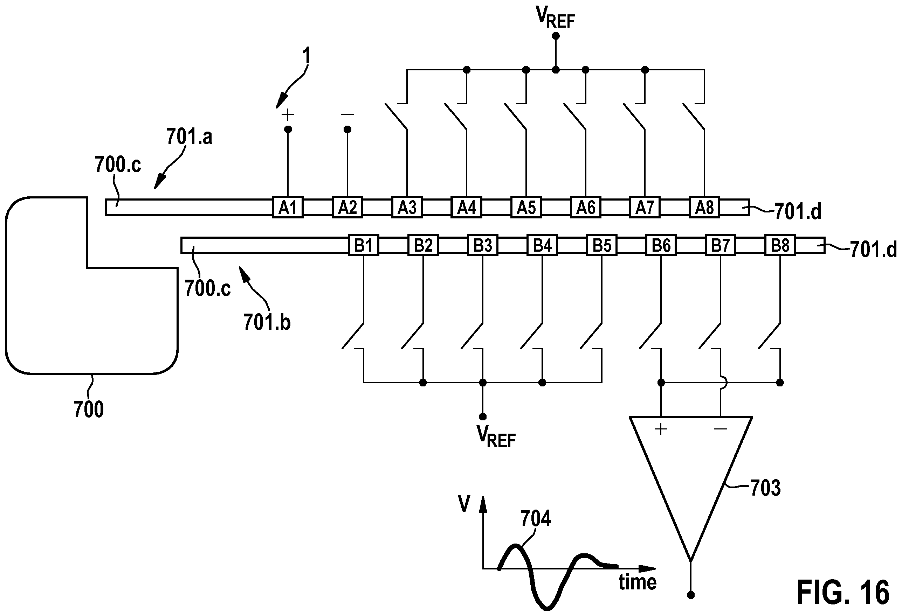

In a preferred further embodiment, the present invention allows automatic determination of the relative positions of inter-lead electrodes based on ECAPs 704 post-stimulus latency value changes. Since the IPG 700 controls both the timing of the stimulation pulse delivery and the sampling of ECAPs 704, it can stimulate at one end of lead 701.a and record at the other end of lead 701.b, thus maximizing the distance between stimulating and recording electrodes, as shown in Figure 16 for a quasi tripolar recording configuration (the same applies to the true tripolar case shown in Figure 15).

In a preferred embodiment, a suitable threshold is defined in an initial ECAP signature and the latency to such threshold calculated. Such latency value directly corresponds to the physical distance between the stimulating and recording electrode sites following implantation and it is stored in the IPG 700 for future comparisons.

During normal operation, the IPG 700 will occasionally initiate an ECAP 704 as in the above-described embodiment. To determine the new ECAP 704 latency, the output of the ECAP recording front-end 703 is compared against the threshold defined using the initial ECAP. Since no subsequent ECAP measurements are required following the initial one, the power and memory required for the purpose of determining relative leads migration is minimized.

If the initial and subsequent latency values are within some acceptable deviation, then it can be assumed leads 701.a and 701.b have not migrated relative to each other or that they have migrated an acceptable small amount. An unacceptable deviation may, for example, be defined as an abrupt or significant short-term change from the initial latency value. It can be assumed latency changes due to factors that affect nerve conduction velocity (e.g. drugs) change relatively slowly, whereas relative migration is more likely to cause an abrupt change in latency values. Accordingly, the latency values may include non-trended calculations, where the initial value does not change over time, and trended measurements, where the initial latency value is adjusted so as to account for slow-varying nerve conduction velocity changes.

In an alternative embodiment, a train of stimulation pulses is delivered following implantation and the corresponding initial ECAPs 704 averaged. This may be required to improve the signal-to-noise ratio in some applications. Similarly, a threshold for latency determination is defined from the averaged initial ECAP 704 signature and an initial latency calculated. Again, during normal operation, the IPG 700 will occasionally initiate a train of ECAPs 704 as in the above-described embodiment. To determine the new ECAP 704 latency, all outputs of the ECAP recording front-end 703 are averaged and compared against the threshold defined using the initial averaged ECAP 704.

Upon detection of an unacceptable latency deviation, the IPG 700 can dynamically alter the electrical stimulation of the target (e.g. the electrodes or current steering settings) that results in a confirmed effectiveness, by ECAPs 704 or the patient, of the modified therapy. In addition, or as an alternative, the IPG 700 has the capability of notifying a physician of the migration, via a remote reporting feature, for further corrective action.

The timing resolution required for detecting lead migration based on ECAP 704 latency changes can be estimated as follows. A large percentage of SCS patients implanted with dual parallel leads experience a mean relative micro migration of approximately 2-3 mm of stagger between their leads, equivalent to one typical SCS electrode offset (cf. Heller "Lead Migration After SCS a 'Universal Problem'", Anesthesiology News, Pain Medicine, vol. 35:5, May 2009). The $A\beta$ fibers recruited by SCS have typical conduction velocities in the range of 30 to 70 m/s. If the target is for example to detect 0.2 mm relative changes, this implies a resolution better than 3.0 $\mu$s which imposes a demanding requirement for sampling in the IPG 700 as most digitalization of biosignals (and other internal signals for operation, e.g. battery measurements) is usually done with 10 bits of resolution at a maximum sampling frequency of 100 kS/s.

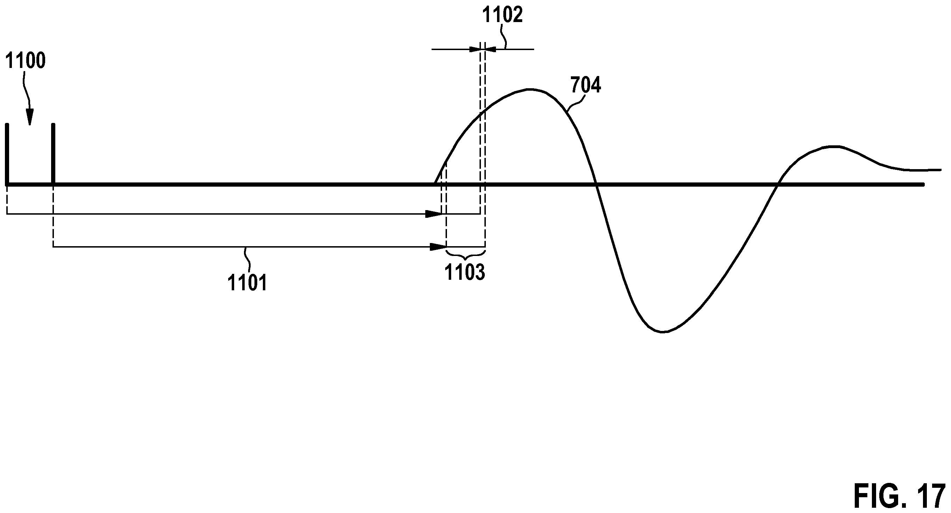

In a preferred further embodiment, as shown in Figure 17, a train of stimulation pulses 1100 is delivered following implantation and each initial ECAP 704 for each pulse sampled with an stimulus-to-start-of-sample timing 1101, or blanking-to-start-of sample timing, incremented a small delta 1102 (less than the sample period). This equivalent-time-sampling (ETS) technique can then be applied to reconstruct the initial ECAP 704 signature with a high degree of temporal accuracy utilizing a reduced sampling frequency (period 1103). As an example, if twenty pulses are used to reconstruct the ECAP 704 and delta 1102 is selected equal to 2.0 $\mu$s, this requires a sampling frequency in the order of 25 kHz (period 1103 40 $\mu$s) which is below the typical time-base of 32,768 Hz typically used in IPGs 700. ETS can be applied since ECAPs 704 can be considered a repetitive signal in the time period required to deliver the ECAP-generating stimulation train 1100. Similar to the previous embodiments, a threshold for latency determination is defined from the reconstructed initial ECAP signature 704 and an initial latency calculated.

During normal operation, the IPG 700 will occasionally initiate a train of ECAPs 704 as in the above-described embodiment. Using the ECAPs 704 generated by such train, a high-resolution ECAP 704 is reconstructed using ETS. To estimate the new latency between stimulating and recording sites, the time where the reconstructed ECAP 704 crosses the selected threshold is determined.

As in previous embodiments, if the initial and subsequent latency values are within some acceptable deviation, then it can be assumed leads 701.a and 701.b have not migrated relative to each other or that they have migrated an acceptable small amount. Latency values may include non-trended calculations, where the initial value does not change over time, and trended measurements, where the initial latency value is adjusted so as to account for slow-varying nerve conduction velocity changes.

Also as in previous embodiments, upon detection of an unacceptable deviation, the IPG 700 can dynamically alter the therapy (e.g. the electrodes or current steering settings) that results in a confirmed effectiveness, by ECAPs 704 or the patient, of the modified therapy. In addition, or as an alternative, the IPG 700 can notify the physician of the migration, via a remote reporting feature, for further corrective action.

Summarizing, the technical advantages of the systems and methods of the present invention used for ECAPs recording advantageously include the feature of providing a balance phase for a given stimulation phase in a multi-electrode system that return the electrodes within mV of their open circuit potentials (OCPs) terminating Faradaic reactions caused by stimulation and thus minimizing the stimulus artifact (SA) for evoked compound action potential (ECAP) recording. The methods and systems according to the invention further provide recording configurations for ECAPs, particularly in spinal cord stimulation (SCS) devices, that minimize the pick-up of interfering signals such as remnant SA, electromyographic (EMG) activity caused by nearby muscles and heart activity (ECG). Finally, the systems and methods according to the invention further provide a robust method for determining relative lead migration, particularly in neuro stimulation systems with a plurality of implanted leads. The method can deliver high temporal resolution utilizing reduced sampling rate.

**Claims**

1. Method for automatic charge balancing during electrical stimulation of a target, using a pulse generator (700) having at least one stimulating electrode (W, X), at least one return electrode (Y), and at least one forced return electrode (Z), wherein the at least one stimulation electrode (W, X) and the at least one return electrode are configured to deliver electrical stimulation therapy, wherein each electrode (W, X, Y, Z) is coupled via a DC-blocking capacitor ($C_i$) to a current source (S), a current sink (S'), or a voltage, and where each electrode forms a capacitance ($C_{dli}$) when forming a double layer with adjacent target material, wherein

   - in a determination stage, programmed stimulation current pulses ($I_{Ni}$) and automatically-determined balancing current pulses ($I_{Pi}$) for the respective electrode (i = W, X, Y, Z) are established such that the difference between the circulating balancing current pulse ($I_{Pi}$) minus the respective stimulation current pulse ($I_{Ni}$) through the respective electrode satisfy:

      - for the at least one stimulating electrode (W, X), the difference equals an automatically determined positive value;
      - for the at least one return electrode (Y) and the at least one forced return electrode (Z), the difference is positive and smaller or equal than the difference value for the stimulation electrode or, in case of more than one stimulation electrode, the minimum among the difference values for the stimulating electrodes (W, X);
      - for each electrode (W, X, Y, Z), both its associated DC-blocking capacitor ($C_i$) and the associated capacitance ($C_{dli}$) charge in the same direction and opposite for stimulating and returning electrodes, and wherein

      - in a stimulation stage succeeding said determination stage, programmed stimulation current pulses ($I_{Ni}$) followed by automatically-determined balancing current pulses ($I_{Pi}$) are repeatedly applied via the at least one stimulating electrode (i = W, X) and at least one return electrode, wherein between a balancing current pulse ($I_{Pi}$) and the next stimulation current pulse ($I_{Ni}$) an open circuit phase (OCP) is conducted where no current is imposed via the at least one stimulating electrode (W, X), and wherein

      - in said stimulation stage at least one of the electrodes (W, X, Y, Z) is monitored, wherein, when an accumulated voltage ($\Delta V_{dli}$) at the double layer of the at least one monitored electrode crosses pre-defined thresholds ($-\Delta V_{AddOCP}$, $\Delta V_{SubOCP}$), correction currents ($I_{CORRStim}$, $I_{CORRRet}$) are generated that reduce or cancel said accumulated voltages ($\Delta V_{dli}$).

2. Method according to claim 1, wherein crossing of the thresholds ($-\Delta V_{AddOCP}$, $\Delta V_{SubOCP}$) by said accumulated double layer voltage ($\Delta V_{dli}$) is automatically detected by comparing the voltage on the terminal of the DC-blocking capacitor ($C_i$) opposite to the electrode against the difference between an internally-generated voltage reference and an estimated accumulated voltage ($P\Delta V_{CStim}|^{Per\ Pulse}$, $P\Delta V_{CRet}|^{Per\ Pulse}$, or $P\Delta V_{CFor}|^{Per\ Pulse}$) at the DC blocking capacitor ($C_i$).

3. Method according to claim 1 or 2, wherein said automatically-determining of balancing current pulses is performed for different patient postures and/or depending on the stimulation frequency.

4. Method according to one of the preceding claims, wherein automatically-determining of balancing current pulses involves letting each electrode for delivering electrical stimulation therapy stimulate against a reference electrode (201), wherein particularly said reference electrode (201) is formed by a casing (201) of the pulse generator (700).

5. Method according to one of the preceding claims, wherein automatically-determining of balancing current pulses is performed such that the stimulus artifact (SA) of a biphasic pulse for evoked compound action potential (ECAP) sensing is minimized.

6. Method according to claim 5, wherein ECAPs are sensed using a tripolar or quasi tripolar arrangement of three recording electrodes (B6, B7, B8) on at least an implantable lead connected to the pulse generator (700).

7. Method according to one of the claims 5 or 6, wherein the pulse generator (700) comprises a first and a second lead (701.a, 701.b) extending along each other from a first end (701.c) of the respective lead to a second end (701.d) of the respective lead, wherein the at least one stimulating electrode (A3, B2) is formed by one of said leads (701.a, 701.b) and guarded by at least one return electrode (A2, A4, B1, B3), and wherein said recording electrodes (B6, B7, B8) are formed by one of said leads (701.a, 701.b).

8. System for automatic charge balancing during electrical stimulation of a target, wherein

- the system (1) comprises a pulse generator (700) having at least one stimulating electrode (W, X), at least one return electrode (Y), and at least one forced return electrode (Z), wherein the at least one stimulation electrode (W, X) and the at least one return electrode are configured to deliver electrical stimulation therapy, wherein each electrode (W, X, Y, Z) is coupled via a DC-blocking capacitor ($C_i$) to a current source (S), a current sink (S'), or a voltage, and where each electrode forms a capacitance ($C_{dli}$) when forming a double layer with adjacent target material, wherein
- the system (1) is configured to program in a determination stage stimulation current pulses ($I_{Ni}$) and automatically-determined balancing current pulses ($I_{Pi}$) for the respective electrode (i = W, X, Y, Z) such that the difference between the circulating balancing current pulse ($I_{Pi}$) minus the respective stimulation current pulse ($I_{Ni}$) through the respective electrode satisfy:

- for the at least one stimulating electrode (W, X), the difference equals an automatically determined positive value;
- for the at least one return electrode (Y) and the at least one forced return electrode (Z), the difference is positive and smaller or equal than the difference value for the stimulation electrode or, in case of more than one stimulation electrode, the minimum among the difference values for the stimulating electrodes (W, X);
- for each electrode (W, X, Y, Z), both its associated DC-blocking capacitor ($C_i$) and the associated capacitance ($C_{dli}$) charge in the same direction and opposite for stimulating and returning electrodes, and wherein

- the system (1) is further configured to repeatedly inject in a stimulation stage succeeding said determination stage, programmed stimulation current pulses ($I_{Ni}$) followed by automatically-determined balancing current pulses ($I_{Pi}$) via the at least one stimulating electrode (i = W, X) and at least one return electrode, wherein between a balancing current pulse ($I_{Pi}$) and the next stimulation current pulse ($I_{Ni}$) an open circuit phase is conducted where no current is imposed via the at least one stimulating electrode (W, X), and wherein

- the system (1) is configured to monitor in said stimulation stage at least one of the electrodes (W, X, Y, Z), wherein, when an accumulated voltage ($\Delta V_{dli}$) at the double layer of the at least one monitored electrode crosses pre-defined thresholds ($-\Delta V_{AddOCP}$, $\Delta V_{SubOCP}$), correction currents ($I_{CORRStim}$, $I_{CORRRet}$) are generated that reduce or cancel said accumulated voltages ($\Delta V_{dli}$).

9. System according to claim 8, wherein the system (1) is configured to detect crossing of the thresholds ($-\Delta V_{AddOCP}$, $\Delta V_{SubOCP}$) by said accumulated double layer voltage ($\Delta V_{dli}$) automatically by comparing the voltage on the terminal of the DC-blocking capacitor ($C_i$) opposite to the electrode against the difference between an internally-generated voltage reference and an estimated accumulated voltage ($P\Delta V_{CStim}|^{Per\ Pulse}$, $P\Delta V_{CRet}|^{Per\ Pulse}$, or $P\Delta V_{CFor}|^{Per\ Pulse}$) at the DC blocking capacitor ($C_i$).

10. System according to claim 8 or 9, wherein the system (1) is configured to perform automatically-determining of balancing current pulses for different patient postures and/or depending on the stimulation frequency.

11. System according to one of the claims 8 to 10, wherein for automatically-determining of balancing current pulses, the system (1) is configured to let each electrode for delivering electrical stimulation therapy stimulate against a reference electrode (201), wherein particularly said reference electrode (201) is formed by a casing (201) of the pulse generator (700).

12. System according to one of the claims 8 to 11, wherein for automatically-determining of balancing current pulses, the system (1) is configured such that the stimulus artifact (SA) of a biphasic pulse for evoked compound action potential (ECAP) sensing is minimized.

13. System according to claim 12, wherein the system (1) further comprises three recording electrodes (B6, B7, B8) on at least an implantable lead connected to the pulse generator (700) that are arranged in a quasi tripolar or a tripolar configuration for measuring ECAPs.

14. System according to one of the claims 8 to 13, wherein the pulse generator (700) comprises a first and a second lead (701.a, 701.b) extending along each other from a first end (701.c) of the respective lead to a second end (701.d) of the respective lead, wherein the at least one stimulating electrode (A3, B2) is formed by one of said leads (701.a, 701.b) and guarded by at least one return electrode (A2, A4, B1, B3), and wherein said recording electrodes (B6, B7, B8) are formed by one of said leads (701.a, 701.b).

15. System according to one of the claims 8 to 14, wherein the current sources (S) or sinks (S') are coupled to the respective electrode (W, X, Y, Z) merely via the respective DC-blocking capacitor ($C_i$).

**Patentansprüche**

1. Verfahren für automatischen Ladungsausgleich während einer elektrischen Stimulation eines Ziels, unter Verwendung eines Impulsgenerators (700) mit mindestens einer Stimulationselektrode (W, X), mindestens einer Gegenelektrode (Y) und mindestens einer Zwangsgegenelektrode (Z), wobei die mindestens eine Stimulationselektrode (W, X) und die mindestens eine Gegenelektrode so eingerichtet sind, dass sie eine elektrische Stimulationstherapie abgeben, wobei jede Elektrode (W, X, Y, Z) über einen Gleichstrom sperrenden Kondensator ($C_i$) mit einer Stromquelle (S), einer Stromsenke (S') oder einer Spannung verbunden ist, und wobei jede Elektrode einen Kondensator ($C_{dli}$) bildet, wenn sie eine Doppelschicht mit angrenzendem Zielmaterial bildet, wobei

- in einer Bestimmungsstufe programmierte Stimulationsstromimpulse ($I_{Ni}$) und automatisch bestimmte Ausgleichsstromimpulse ($I_{Pi}$) für die jeweilige Elektrode (i = W, X, Y, Z) derart ermittelt werden, dass die Differenz zwischen dem durchfließenden Ausgleichsstromimpuls ($I_{Pi}$) abzüglich des jeweiligen Stimulationsstromimpulses ($I_{Ni}$) durch die jeweilige Elektrode Folgendes erfüllt:

- für die mindestens eine Stimulationselektrode (W, X) entspricht die Differenz einem automatisch bestimmten positiven Wert;
- für die mindestens eine Gegenelektrode (Y) und die mindestens eine Zwangsgegenelektrode (Z) ist die Differenz positiv und kleiner oder gleich dem Differenzwert für die Stimulationselektrode oder, bei mehr als einer Stimulationselektrode, dem Minimum unter den Differenzwerten für die Stimulationselektroden (W, X);
- für jede Elektrode (W, X, Y, Z) laden sich sowohl der zugehörige Gleichstrom sperrende Kondensator ($C_i$) als auch der zugehörige Kondensator ($C_{dli}$) in derselben Richtung und für Stimulations- und Gegenelektrode entgegengesetzt auf, und wobei

- in einer Stimulationsstufe im Anschluss an die Bestimmungsstufe programmierte Stimulationsstromimpulse ($I_{Ni}$), gefolgt von automatisch bestimmten Ausgleichsstromimpulsen ($I_{Pi}$) wiederholt über die mindestens eine Stimulationselektrode (i = W, X) und mindestens eine Gegenelektrode ausgesendet werden, wobei zwischen einem Ausgleichsstromimpuls ($I_{Pi}$) und dem nächsten Stimulationsstromimpuls ($I_{Ni}$) eine Phase eines offenen Schaltkreises (OCP) durchgeführt wird, in der über die mindestens eine Stimulationselektrode (W, X) kein Strom eingeprägt wird, und wobei

- in der Stimulationsstufe mindestens eine der Elektroden (W, X, Y, Z) überwacht wird, wobei, wenn eine akkumulierte Spannung ($\Delta V_{dli}$) an der Doppelschicht der mindestens einen überwachten Elektrode vorher festgelegte Schwellenwerte ($-\Delta V_{AddOCP}$, $\Delta V_{SubOCP}$) überschreitet, Korrekturströme ($I_{CORRStim}$, $I_{CORRRet}$) erzeugt werden, die die akkumulierten Spannungen ($\Delta V_{dli}$) reduzieren oder ausgleichen.

2. Verfahren nach Anspruch 1, wobei ein Überschreiten der Schwellenwerte ($-\Delta V_{AddOCP}$, $\Delta V_{SubOCP}$) durch die akkumulierte Doppelschichtspannung ($\Delta V_{dli}$) durch Vergleichen der Spannung an dem Anschluss des Gleichstrom sperrenden Kondensators ($C_i$) entgegengesetzt zur Elektrode mit der Differenz zwischen einer intern erzeugten Bezugsspannung und einer geschätzten akkumulierten Spannung ($P\Delta V_{CStim}|^{Per\ Pulse}$, $P\Delta V_{CRet}|^{Per\ Pulse}$ oder $P\Delta V_{CFor}|^{PerPulse}$) am Gleichstrom sperrenden Kondensator ($C_i$) automatisch erfasst wird.

3. Verfahren nach Anspruch 1 oder 2, wobei das automatische Bestimmen von Ausgleichsstromimpulsen für unterschiedliche Körperhaltungen von Patienten und/oder je nach Stimulationsfrequenz durchgeführt wird.

4. Verfahren nach einem der vorhergehenden Ansprüche, wobei das automatische Bestimmen von Ausgleichsstromimpulsen beinhaltet, dass jede Elektrode für die Abgabe einer elektrischen Stimulationstherapie gegen eine Bezugselektrode (201) stimuliert, wobei insbesondere die Bezugselektrode (201) von einem Gehäuse (201) des Impulsgenerators (700) gebildet wird.

5. Verfahren nach einem der vorhergehenden Ansprüche, wobei das automatische Bestimmen von Ausgleichsstromimpulsen derart durchgeführt wird, dass das Reizartefakt (SA) eines zweiphasigen Impulses für die Erfassung von evozierten Summenaktionspotenzialen (ECAP) minimiert wird.

6. Verfahren nach Anspruch 5, wobei ECAP unter Verwendung einer dreipoligen oder quasi dreipoligen Anordnung von drei Aufzeichnungselektroden (B6, B7, B8) an mindestens einer implantierbaren Leitung erfasst werden, die mit dem Impulsgenerator (700) verbunden ist.

7. Verfahren nach Anspruch 5 oder 6, wobei der Impulsgenerator (700) eine erste und eine zweite Leitung (701.a, 701.b) umfasst, die von einem ersten Ende (701.c) der jeweiligen Leitung zu einem zweiten Ende (701.d) der jeweiligen Leitung aneinander entlang verlaufen, wobei die mindestens eine Stimulationselektrode (A3, B2) von einer der Leitungen (701.a, 701.b) gebildet und von mindestens einer Gegenelektrode (A2, A4, B1, B3) geschützt wird, und wobei die Aufzeichnungselektroden (B6, B7, B8) von einer der Leitungen (701.a, 701.b) gebildet werden.

8. System für automatischen Ladungsausgleich währen einer elektrischen Stimulation eines Ziels, wobei

   - das System (1) einen Impulsgenerator (700) mit mindestens einer Stimulationselektrode (W, X), mindestens einer Gegenelektrode (Y) und mindestens einer Zwangsgegenelektrode (Z) umfasst, wobei die mindestens eine Stimulationselektrode (W, X) und die mindestens eine Gegenelektrode so eingerichtet sind, dass sie eine elektrische Stimulationstherapie abgeben, wobei jede Elektrode (W, X, Y, Z) über einen Gleichstrom sperrenden Kondensator ($C_i$) mit einer Stromquelle (S), einer Stromsenke (S') oder einer Spannung verbunden ist, und wobei jede Elektrode einen Kondensator ($C_{dli}$) bildet, wenn sie eine Doppelschicht mit angrenzendem Zielmaterial bildet, wobei
   - das System (1) so eingerichtet ist, dass es in einer Bestimmungsstufe Stimulationsstromimpulse ($I_{Ni}$) und automatisch bestimmte Ausgleichsstromimpulse ($I_{Pi}$) für die jeweilige Elektrode (i = W, X, Y, Z) derart programmiert, dass die Differenz zwischen dem durchfließenden Ausgleichsstromimpuls ($I_{Pi}$) abzüglich des jeweiligen Stimulationsstromimpulses ($I_{Ni}$) durch die jeweilige Elektrode Folgendes erfüllt:
   - für die mindestens eine Stimulationselektrode (W, X) entspricht die Differenz einem automatisch bestimmten positiven Wert;
   - für die mindestens eine Gegenelektrode (Y) und die mindestens eine Zwangsgegenelektrode (Z) ist die Differenz positiv und kleiner oder gleich dem Differenzwert für die Stimulationselektrode oder, bei mehr als einer Stimulationselektrode, dem Minimum unter den Differenzwerten für die Stimulationselektroden (W, X);
   - für jede Elektrode (W, X, Y, Z) laden sich sowohl der zugehörige Gleichstrom sperrende Kondensator ($C_i$) als auch der zugehörige Kondensator ($C_{dli}$) in derselben Richtung und für Stimulations- und Gegenelektrode entgegengesetzt auf, und wobei

   - das System (1) ferner so eingerichtet ist, dass es in einer Stimulationsstufe im Anschluss an die Bestimmungsstufe programmierte Stimulationsstromimpulse ($I_{Ni}$), gefolgt von automatisch bestimmten Ausgleichsstromimpulsen ($I_{Pi}$), wiederholt über die mindestens eine Stimulationselektrode (i = W, X) und mindestens eine Gegenelektrode einspeist, wobei zwischen einem Ausgleichsstromimpuls ($I_{Pi}$) und dem nächsten Stimulationsstromimpuls ($I_{Ni}$) eine Phase eines offenen Schaltkreises (OCP) durchgeführt wird, in der über die mindestens eine Stimulationselektrode (W, X) kein Strom eingeprägt wird, und wobei

   - das System (1) so eingerichtet ist, dass es in der Stimulationsstufe mindestens eine der Elektroden (W, X, Y, Z) überwacht, wobei, wenn eine akkumulierte Spannung ($\Delta V_{dli}$) an der Doppelschicht der mindestens einen überwachten Elektrode vorher festgelegte Schwellenwerte ($-\Delta V_{AddOCP}$, $\Delta V_{SubOCP}$) überschreitet, Korrekturströme ($I_{CORRStim}$, $I_{CORRRet}$) erzeugt werden, die die akkumulierten Spannungen ($\Delta V_{dli}$) reduzieren oder ausgleichen.

**9.** System nach Anspruch 8, wobei das System (1) so eingerichtet ist, dass es ein Überschreiten der Schwellenwerte ($-\Delta V_{AddOCP}$, $\Delta V_{SubOCP}$) durch die akkumulierte Doppelschichtspannung ($\Delta V_{dli}$) automatisch erfasst durch Vergleichen der Spannung an dem Anschluss des Gleichstrom sperrenden Kondensators ($C_i$) entgegengesetzt zur Elektrode mit der Differenz zwischen einer intern erzeugten Bezugsspannung und einer geschätzten akkumulierten Spannung ($P\Delta V_{CStim}|^{Per\ Pulse}$, $P\Delta V_{CRet}|^{Per\ Pulse}$ oder $P\Delta V_{CFor}|^{PerPulse}$) am Gleichstrom sperrenden Kondensator ($C_i$).

**10.** System nach Anspruch 8 oder 9, wobei das System (1) so eingerichtet ist, dass es das automatische Bestimmen von Ausgleichsstromimpulsen für unterschiedliche Körperhaltungen von Patienten und/oder je nach Stimulationsfrequenz durchführt.

**11.** System nach einem der Ansprüche 8 bis 10, wobei zum automatischen Bestimmen von Ausgleichsstromimpulsen das System (1) so eingerichtet ist, dass es jede Elektrode für die Abgabe einer elektrischen Stimulationstherapie gegen eine Bezugselektrode (201) stimulieren lässt, wobei insbesondere die Bezugselektrode (201) von einem Gehäuse (201) des Impulsgenerators (700) gebildet wird.

**12.** System nach einem der Ansprüche 8 bis 11, wobei zum automatischen Bestimmen von Ausgleichsstromimpulsen das System (1) so eingerichtet ist, dass das Reizartefakt (SA) eines zweiphasigen Impulses für die Erfassung von evozierten Summenaktionspotenzialen (ECAP) minimiert wird.

**13.** System nach Anspruch 12, wobei das System (1) ferner drei Aufzeichnungselektroden (B6, B7, B8) an mindestens einer implantierbaren Leitung umfasst, die mit dem Impulsgenerator (700) verbunden ist, die in einer quasi dreipoligen oder dreipoligen Gestaltung zum Messen von ECAP angeordnet sind.

**14.** System nach einem der Ansprüche 8 bis 13, wobei der Impulsgenerator (700) eine erste und eine zweite Leitung (701.a, 701.b) umfasst, die von einem ersten Ende (701.c) der jeweiligen Leitung zu einem zweiten Ende (701.d) der jeweiligen Leitung aneinander entlang verlaufen, wobei die mindestens eine Stimulationselektrode (A3, B2) von einer der Leitungen (701.a, 701.b) gebildet und von mindestens einer Gegenelektrode (A2, A4, B1, B3) geschützt ist, und wobei die Aufzeichnungselektroden (B6, B7, B8) von einer der Leitungen (701.a, 701.b) gebildet sind.

**15.** System nach einem der Ansprüche 8 bis 14, wobei die Stromquellen (S) oder - senken (S') mit der jeweiligen Elektrode (W, X, Y, Z) lediglich über den jeweiligen Gleichstrom sperrenden Kondensator ($C_i$) verbunden sind.

## Revendications

**1.** Procédé d'équilibrage de charge automatique durant une stimulation électrique d'une cible en utilisant un générateur d'impulsions (700) ayant au moins une électrode de stimulation (W, X), au moins une électrode de retour (Y) et au moins une électrode de retour forcé (Z), où l'au moins une électrode de stimulation (W,X) et l'au moins une électrode de retour sont configurées pour administrer une thérapie de stimulation électrique, où chaque électrode (W, X, Y, Z) est couplée à une source de courant (S), un puits de courant (S') ou une tension, par le biais d'un condensateur de blocage de courant continu ($C_i$), et où chaque électrode forme une capacité électrique ($C_{dli}$) lors de la formation d'une double couche avec un matériau cible adjacent, où

- dans un stade de détermination, des impulsions de courant de stimulation programmées ($I_{Ni}$) et des impulsions de courant d'équilibre déterminées automatiquement ($I_{Pi}$) pour l'électrode respective (i = W, X, Y, Z) sont établies de sorte que la différence entre l'impulsion de courant d'équilibre en circulation ($I_{Pi}$) diminuée de l'impulsion de courant de stimulation respective ($I_{Ni}$) par l'électrode respective satisfait :

- pour l'au moins une électrode de stimulation (W, X), à ce que la différence est égale à une valeur positive déterminée automatiquement ;
- pour l'au moins une électrode de retour (Y) et l'au moins une électrode de retour forcé (Z), à ce que la différence est positive et inférieure ou égale à la valeur de la différence pour l'électrode de stimulation, ou, dans le cas de plus d'une électrode de stimulation, au minimum parmi les valeurs de différence pour les électrodes de stimulation (W, X) ;
- pour chaque électrode (W, X, Y, Z), à la fois son condensateur à blocage de courant continu ($C_i$) associé et la capacité ($C_{dli}$) associée chargent dans la même direction et en opposition pour les électrodes de stimulation et de retour, et où

    - dans un stade de stimulation succédant audit stade de détermination, des impulsions de courant de stimulation programmées ($I_{Ni}$) suivies d'impulsions de courant d'équilibre déterminées automatiquement ($I_{Pi}$) sont appliquées de manière répétée par le biais de l'au moins une électrode de stimulation ($i = W, X$) et l'au moins une électrode de retour, où, entre une impulsion de courant d'équilibre ($I_{Pi}$) et l'impulsion de courant de stimulation ($I_{Ni}$) suivante, une phase de circuit ouvert (OCP) est réalisée où aucun courant n'est imposé par le biais de l'au moins une électrode de stimulation (W, X), et où
    - dans ledit stade de stimulation, au moins une des électrodes (W, X, Y, Z) est surveillée, où, lorsqu'une tension accumulée ($\Delta V_{dli}$) au niveau de la double couche de l'au moins une électrode surveillée passe par des limites prédéfinies ($-\Delta V_{AddOCP}$, $\Delta V_{SubOCP}$), des courants de correction ($I_{CORRStim}$, $I_{CORRet}$) sont générés qui réduisent ou annulent lesdites tensions accumulées ($\Delta V_{dli}$).

2. Procédé selon la revendication 1, dans lequel le passage des limites ($-\Delta V_{AddOCP}$, $\Delta V_{SubOCP}$) par ladite tension de double couche accumulée ($\Delta V_{dli}$).est automatiquement détecté par la comparaison de la tension sur la borne du condensateur de blocage de courant continu ($C_i$) opposé à l'électrode avec la différence entre une référence de tension générée de manière interne et une tension accumulée estimée $P\Delta V_{CStim} \mid$ $^{Per\ Pulse}$, $P\Delta V_{CRet} \mid$ $^{Per\ Pulse}$, ou $P\Delta V_{CFor} \mid$ $^{Per\ Pulse}$) au niveau du condensateur de blocage de courant continu ($C_i$).

3. Procédé selon la revendication 1 ou 2, dans lequel ladite détermination de manière automatique d'impulsions de courant d'équilibre est effectuée pour différentes postures du patient et/ou en fonction de la fréquence de stimulation.

4. Procédé selon l'une des revendications précédentes, dans lequel la détermination de manière automatique d'impulsions de courant d'équilibre implique le fait de laisser chaque électrode administrer des stimulations de thérapie de stimulation électrique par rapport à une électrode de référence (201), où ladite électrode de référence (201) est particulièrement formée par une cage (201) du générateur d'impulsions (700).

5. Procédé selon l'une des revendications précédentes, dans lequel la détermination de manière automatique d'impulsions de courant d'équilibre est effectuée de sorte que l'artéfact de stimulation (SA) d'une impulsion biphasique pour une détection d'un potentiel d'action composé évoqué (ECAP) est minimisé.

6. Procédé selon la revendication 5, dans lequel les potentiels ECAP sont détectés en utilisant un agencement tripolaire ou quasi tripolaire de trois électrodes d'enregistrement (B6, B7, B8) sur au moins un conducteur implantable connecté au générateur d'impulsions (700).

7. Procédé selon l'une des revendications 5 ou 6, dans lequel le générateur d'impulsions (700) comprend un premier et un second conducteur (701.a, 701.b) s'étendant l'un le long de l'autre à partir d'une première extrémité (701.c) du conducteur respectif à une seconde extrémité (701.d) du conducteur respectif, où l'au moins une électrode de stimulation (A3, B2) est formée par l'un desdits conducteurs (701.a, 701.b) et est protégée par au moins une électrode de retour (A2, A4, B1, B3), et où lesdites électrodes d'enregistrement (B6, B7, B8) sont formées par l'un desdits conducteurs (701.a, 701.b).

8. Système prévu pour un équilibrage de charge automatique durant une stimulation électrique d'une cible, dans lequel

    - le système (1) comprend un générateur d'impulsions (700) ayant au moins une électrode de stimulation (W, X), au moins une électrode de retour (Y), et au moins une électrode de retour forcé (Z), où l'au moins une électrode de stimulation (W,X) et l'au moins une électrode de retour sont configurées pour administrer une thérapie de stimulation électrique, où chaque électrode (W, X, Y, Z) est couplée à une source de courant (S), un puits de courant (S') ou une tension, par le biais d'un condensateur de blocage de courant continu ($C_i$), et où chaque électrode forme une capacité électrique ($C_{dli}$) lors de la formation d'une double couche avec un matériau cible adjacent, où
    - le système (1) est configuré pour programmer, dans un stade de détermination, des impulsions de courant de stimulation ($I_{Ni}$) et des impulsions de courant d'équilibre déterminées automatiquement ($I_{Pi}$) pour l'électrode respective ($i = W, X, Y, Z$), de sorte que la différence entre l'impulsion de courant d'équilibre en circulation ($I_{Pi}$) diminuée de l'impulsion de courant de stimulation respective ($I_{Ni}$) par l'électrode respective satisfasse :

- pour l'au moins une électrode de stimulation (W, X), à ce que la différence soit égale à une valeur positive déterminée automatiquement ;
- pour l'au moins une électrode de retour (Y) et l'au moins une électrode de retour forcé (Z), à ce que la différence soit positive et inférieure ou égale à la valeur de la différence pour l'électrode de stimulation, ou, dans le cas de plus d'une électrode de stimulation, au minimum parmi les valeurs de différence pour les électrodes de stimulation (W, X) ;
- pour chaque électrode (W, X, Y, Z), à la fois leur condensateur à blocage de courant continu ($C_i$) associé et la capacité ($C_{dli}$) associée chargent dans la même direction et en opposition pour les électrodes de stimulation et de retour, et où

- le système (1) est en outre configuré pour injecter de manière répétée, dans un stade de stimulation succédant audit stade de détermination, des impulsions de courant de stimulation programmées ($I_{Ni}$) suivies d'impulsions de courant d'équilibre déterminées automatiquement ($I_{Pi}$) par le biais de l'au moins une électrode de stimulation (i = W, X) et l'au moins une électrode de retour, où, entre une impulsion de courant d'équilibre ($I_{Pi}$) et l'impulsion de courant de stimulation ($I_{Ni}$) suivante, une phase de circuit ouvert est réalisée où aucun courant n'est imposé par le biais de l'au moins une électrode de stimulation (W, X), et où

- le système (1) est configuré pour surveiller, dans ledit stade de stimulation, au moins une des électrodes (W, X, Y, Z), où, lorsqu'une tension accumulée ($\Delta V_{dli}$) au niveau de la double couche de l'au moins une électrode surveillée passe par des limites prédéfinies ($-\Delta V_{AddOCP}$, $\Delta V_{SubOCP}$), des courants de correction ($I_{CORRStim}$, $I_{CORRet}$) sont générés qui réduisent ou annulent lesdites tensions accumulées ($\Delta V_{dli}$).

9. Système selon la revendication 8, où le système (1) est configuré pour détecter automatiquement le passage des limites ($-\Delta V_{AddOCP}$, $\Delta V_{subOCP}$) par ladite tension de double couche accumulée ($\Delta V_{dli}$). par la comparaison de la tension sur la borne du condensateur de blocage de courant continu ($C_i$) opposé à l'électrode avec la différence entre une référence de tension générée de manière interne et une tension accumulée estimée $P\Delta V_{CStim}|^{Per\ Pulse}$, $P\Delta V_{CRet}|^{Per\ Pulse}$, ou $P\Delta V_{CFor}|^{Per\ Pulse}$) au niveau du condensateur de blocage de courant continu ($C_i$).

10. Système selon la revendication 8 ou 9, où le système (1) est configuré pour effectuer une détermination de manière automatique d'impulsions de courant d'équilibre pour différentes postures du patient et/ou en fonction de la fréquence de stimulation.

11. Système selon l'une des revendications 8 à 10, dans lequel, pour la détermination de manière automatique d'impulsions de courant d'équilibre, le système (1) est configuré pour laisser chaque électrode administrer des stimulations de thérapie de stimulation électrique par rapport à une électrode de référence (201), où ladite électrode de référence (201) est particulièrement formée par une cage (201) du générateur d'impulsions (700).

12. Système selon l'une des revendications 8 à 11, dans lequel, pour la détermination de manière automatique d'impulsions de courant d'équilibre, le système (1) est configuré de sorte que l'artéfact de stimulation (SA) d'une impulsion biphasique pour une détection d'un potentiel d'action composé évoqué (ECAP) est minimisé.

13. Système selon la revendication 12, où le système (1) comprend en outre trois électrodes d'enregistrement (B6, B7, B8) sur au moins un conducteur implantable connecté au générateur d'impulsions (700) qui sont agencées dans une configuration quasi tripolaire ou tripolaire pour la mesure des potentiels ECAP.

14. Système selon l'une des revendications 8 à 13, dans lequel le générateur d'impulsions (700) comprend un premier et un second conducteur (701.a, 701.b) s'étendant l'un le long de l'autre à partir d'une première extrémité (701.c) du conducteur respectif jusqu'à une seconde extrémité (701.d) du conducteur respectif, où l'au moins une électrode de stimulation (A3, B2) est formée par l'un desdits conducteurs (701.a, 701.b) et est protégée par au moins une électrode de retour (A2, A4, B1, B3), et où lesdites électrodes d'enregistrement (B6, B7, B8) sont formées par l'un desdits conducteurs (701.a, 701.b).

15. Système selon l'une des revendications 8 à 14, dans lequel les sources de courant (S) ou les puits de courant (S') sont couplés à l'électrode respective (W, X, Y, Z) simplement par le condensateur de blocage de courant continu ($C_i$) respectif.

FIG. 1

EP 3 216 489 B1

**FIG. 2**

EP 3 216 489 B1

Stimulation Phase

Balance Phase

700

700

EP 3 216 489 B1

FIG. 3

FIG. 4

FIG. 5

$V_I^*$

$V_i^*$

$V_N^*$

MUX
N:3

601

AMP

+

−

$V_{iBUF}^*$

## FIG. 6a

$V_1^*$

$V_{Stim}^*$

$V_{Ret}^*$

$V_{For}^*$

$V_N^*$

MUX
N:3

$V_{MUXStim}^*$

601

$V_{MUXRet}^*$

$V_{MUXFor}^*$

## FIG. 6b

FIG. 7

where $\Delta I_Z$ results equal to $I_{DiffW} + I_{DiffX} - \Delta I_Y > 0$

$$\Delta V_{dli} \leq \Delta V_{SubOCP}$$

Per Pulse

$V_{REFStim}\text{-}P\Delta V_{CStim}$

$V^*_{MUXStim}$

801

**FIG. 8**

$$\Delta V_{dli} \leq -\Delta V_{AddOCP}$$

Per Pulse

$V_{REFRet} \text{-}P\Delta V_{CRet}$

$V^*_{MUXRet}$

901

$$\Delta V_{dli} \leq -\Delta V_{AddOCP}$$

Per Pulse

$V_{REFRet} \text{-}P\Delta V_{CFor}$

$V^*_{MUXFor}$

902

**FIG. 9**

**Correction Phase**

FIG. 10

Balance

Correction

0

Compare

Interphase

time

Stimulation

Stimulation

## FIG. 11

$V_{REFFigs} - (P-R)\, \Delta V_{COutput}$

Per Pulse

$\Delta V_{dli} \lesseqgtr 0$

$V^*_{MUXOutput}$

Output = Stim, Ret, For

## FIG. 12

FIG. 13

EP 3 216 489 B1

FIG. 14

EP 3 216 489 B1

FIG. 15

FIG. 16

EP 3 216 489 B1

FIG. 17

EP 3 216 489 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6301505 B1
- US 20110125217 A1
- US 20080015641 A1
- WO 2014134075 A1
- WO 9721324 A1
- WO 2015164418 A1
- US 8419645 B2

### Non-patent literature cited in the description

- Electrochemical Evaluation of Platinum and Diamond Electrodes for Neural Stimulation. **E. HUDAK.** PhD Thesis, Dept. of Chemical Engineering. Case Western University, May 2011
- **A. T. DO ; Y. S. TAN ; G. M. XIONG ; C. CHOONG ; Z. H. KONG ; K. S. YEO.** A current-mode stimulator circuit with two-step charge balancing background calibration. *2013 IEEE International Symposium on Circuits and Systems (ISCAS2013),* 2013, 409-412
- **HELLER.** Lead Migration After SCS a 'Universal Problem. *Anesthesiology News, Pain Medicine,* May 2009, vol. 35, 5